# EUROPEAN PATENT SPECIFICATION

(11) **EP 0 769 940 B1**
(45) Date of publication and mention of the grant of the patent: **17.09.2003**
(21) Application number: 95928104.9
(22) Date of filing: 20.07.1995
(51) Int. Cl.: A61K 31/192, C07C 57/46, A61P 29/00

(54) **INDENE sPLA2 INHIBITORS**
INDENVERBINDUNGEN ALS sPLA2-INHIBITOREN
COMPOSES D'INDENE UTILES POUR INHIBER sPLA2

(30) Priority: 21.07.1994 US 278441
(43) Date of publication of application: 02.05.1997
(73) Proprietor: ELI LILLY AND COMPANY, Indianapolis, Indiana 46285 (US); SHIONOGI & CO., LTD., Osaka 553 (JP)
(72) Inventor: DILLARD, Robert D., Zionsville, IN 46077 (US); HAGISHITA, Sanji, Nara 639-22 (JP); OHTANI, Mitsuaki, Nara 630 (JP)
(74) Representative: Pritchard, Judith
(86) International application number: PCT/US95/09246
(87) International publication number: WO 96/003120

(56) References cited:
- DE-A- 2 202 715
- DE-A- 2 202 727
- FR-A- 1 505 112
- US-A- 3 732 292
- US-A- 3 888 902
- US-A- 3 954 852
- US-A- 5 093 356

## Description

### Field of the Invention

This invention relates to novel indene compounds useful for inhibiting sPLA₂ mediated release of fatty acids for conditions such as septic shock.

### Background of the Invention

The structure and physical properties of human non-pancreatic secretory phospholipase A₂ (hereinafter called, "sPLA₂") has been thoroughly described in two articles, namely, "Cloning and Recombinant Expression of Phospholipase A₂ Present in Rheumatoid Arthritic Synovial Fluid" by Seilhamer, Jeffrey J.; Pruzanski, Waldemar; Vadas Peter; Plant, Shelley; Miller, Judy A.; Kloss, Jean; and Johnson, Lorin K,; The Journal of Biological Chemistry, Vol. 264, No. 10, Issue of April 5, pp. 5335-5338, 1989; and "Structure and Properties of a Human Non-pancreatic Phospholipase A₂" by Kramer, Ruth M.; Hession, Catherine; Johansen, Berit; Hayes, Gretchen; McGray, Paula; Chow, E. Pingchang; Tizard, Richard; and Pepinsky, R. Blake; The Journal of Biological Chemistry, Vol. 264, No. 10, Issue of April 5, pp. 5768-5775, 1989.

It is believed that sPLA₂ is a rate limiting enzyme in the arachidonic acid cascade which hydrolyzes membrane phospholipids. Thus, it is important to develop compounds which inhibit sPLA₂ mediated release of fatty acids (e.g., arachidonic acid). Such compounds would be of value in general treatment of conditions induced and/or maintained by overproduction of sPLA₂; such as septic shock, adult respiratory distress syndrome, pancreatitis, trauma, bronchial asthma, allergic rhinitis, rheumatoid arthritis, and etc.

U.S. Patent No. 2,825,734 describes the preparation of 3-(2-amino-1-hydroxyethyl) indoles using 3-indole glyoxylamide intermediates such as 1-phenethyl-2-ethyl-6-carboxy-N-propyl-3-indoleglyoxylamide (see, Example 30).

U.S. Patent No. 2,890,233 describes several amide derivatives of 3-indoleacetic acids.

U.S. Patents No. 3,196,162; 3,242,162; 3,242,163; and 3,242,193 (see, Col. 3, lines 55-60, Example 56) describe indolyl aliphatic acids together with their related esters and amides.

U.S. Patent No. 3,449,363 describes trifluoromethylindoles having glyoxylamide groups at the 3 position of the indole nucleus. These compounds are stated to be analgesics.

U.S. Patent No. 5,132,319 describes certain 1-(hydroxylaminoalkyl)indoles derivatives as inhibitors of leukotriene biosynthesis.

The article, "Structure-activity relationships leading to WAY-121,520, a tris aryl-type, indomethacin-based, phospholipase A₂ (PLA₂)/leukotriene biosynthesis inhibitor", by A Kreft, et. al., Agents and Actions, Special Conference Issue Vol. 39 (1993),pp. C33-C35, ISSN 0065-4299, published by Birkhauser Verlag, Basel Switzerland; (Proceedings of the Sixth International Conference of the Inflammation Research Association, September 20-24, 1992, at White Haven, PA/USA, Guest Editors, D.W. Morgan and A.K. Welton) describes the inhibition of phospholipase A2 by indomethacin analogs. Indole compounds having benzyl and acidic substituents are described.

The article, (Short communication) entitled, "Indolizine derivatives with biological activity VI 1-(2-aminoethyl)-3-benzyl-7-methoxy-2-methylindolizine, benanserin structural analogue" by GM Cingolani, F. Claudi, M. Massi, and F. Venturi, Eur. J. Med. Chem. (1990) 25, pp. 709-712 published by Elsevier, Paris describes selected indolizines and their activity on smooth muscle.

European Patent Application No. 0 519 353 (Application No. 92109968.5) describes indolizin derivatives which have pharmacological activities such as inhibitory activity on testosteron reductase.

European Patent Application No. 0 620 215 describes 1H-indole-3-acetamides having utility as sPLA₂ inhibitors.

It is desirable to develop new compounds and treatments for sPLA₂ induced diseases.

### Summary of the Invention

This invention is a novel use of indene compounds as defined in the claims having the nucleus and substituent numbering shown below: 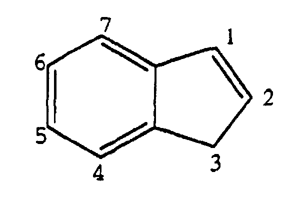 Moreover, the indene compounds of the invention have the general configurations shown in structural formulae "G" below:

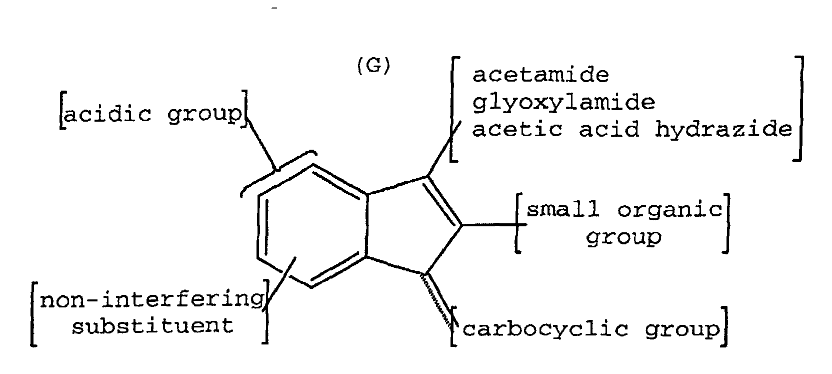

In formula "G" an acetamide, acetic acid hydrazide, or glyoxylamide moiety is present at the 1 position; a large (C₇-C₃₀) organic (e.g., carbocyclic) group is present at the 3 position and is attached to the indene nucleus with a single, or optionally, a double bond; an acidic group is substituted at the 6 or 7 position, and a small organic group is substituted at the 2 position.

This invention is also a pharmaceutical composition containing indene-1-functional compounds as defined in the claims.

This invention is also the use of compounds selected from the group consisting of the novel indene compounds represented by the general formulae "G" as defined in the claims for the manufacture of a medicament for the treatment of septic shock, adult respiratory distress syndrome, pancreatitis, trauma, bronchial asthma, allergic rhinitis, rheumatoid arthritis, and related diseases.

### Detailed Description of the Invention

### Definitions:

The indene acetamides, acetic acid hydrazides (hereinafter called, "hydrazides), and glyoxylamides of the invention employ certain defining terms as follows:

The term, "alkyl" by itself or as part of another substituent means, unless otherwise defined, a straight or branched chain monovalent hydrocarbon radical such as methyl, ethyl, n-propyl, isopropyl, n-butyl, tertiary butyl, isobutyl, sec-butyl, n-pentyl, and n-hexyl.

The term, "alkenyl" employed alone or in combination with other terms means a straight chain or branched monovalent hydrocarbon group having the stated number range of carbon atoms, and typified by groups such as vinyl, propenyl, crotonyl, isopentenyl, and various butenyl isomers.

The term, "hydrocarbyl" means an organic group containing only carbon and hydrogen.

The term, "halo" means fluoro, chloro, bromo, or iodo.

The term, "heterocyclic radical", refers to radicals derived from monocyclic or polycyclic, saturated or unsaturated, substituted or unsubstituted heterocyclic nuclei having 5 to 14 ring atoms and containing from 1 to 3 hetero atoms selected from the group consisting of nitrogen, oxygen or sulfur. Typical heterocyclic radicals are pyrrolyl, furanyl, thiophenyl, pyrazolyl, imidazolyl, phenylimidazolyl, triazolyl, isoxazolyl, oxazolyl, thiazolyl, thiadiazolyl, indolyl, carbazolyl, norharmanyl, azaindolyl, benzofuranyl, dibenzofuranyl, thianaphtheneyl, dibenzothiophenyl, indazolyl, imidazo(1.2-A)pyridinyl, benzotriazolyl, anthranilyl, 1,2-benzisoxazolyl, benzoxazolyl, benzothiazolyl, purinyl, pyridinyl, dipyridinyl, phenylpyridinyl, benzylpyridinyl, pyrimidinyl, phenylpyrimidinyl, pyrazinyl, 1,3,5-triazinyl, quinolinyl, phthalazinyl, quinazolinyl, and quinoxalinyl.

The term, "carbocyclic radical" refers to radicals derived from a saturated or unsaturated, substituted or unsubstituted 5 to 14 membered organic nucleus whose ring forming atoms (other than hydrogen) are solely carbon atoms. Typical carbocyclic radicals are benzylidene, cycloalkyl, cycloalkenyl, phenyl, naphthyl, norbornanyl, bicycloheptadienyl, toluyl, xylenyl, indenyl, stilbenyl, terphenylyl, diphenylethylenyl, phenyl-cyclohexenyl, acenaphthylenyl, and anthracenyl, biphenyl, bibenzylyl and related bibenzylyl homologues represented by the formula (bb), 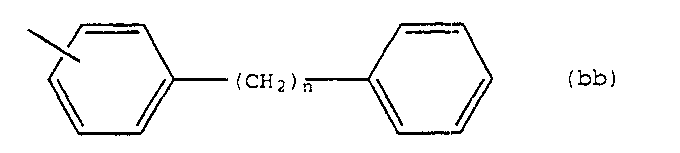 where n is a number from 1 to 8.

The term, "non-interfering substituent", refers to radicals which do not prevent or significantly reduce the inhibition of sPLA₂ mediated release of fatty acids. Non-interfering substituents are suitable for substitution at positions 4, 5, 6, and/or 7 on the indene nucleus (as hereinafter depicted in Formula I) and radical(s) suitable for substitution on the heterocyclic radical and carbocyclic radical as defined above which do not have a significant adverse effect in reducing the inhibiting effect of sPLA₂ mediated release of fatty acids. Illustrative non-interfering radicals are C₁-C₆ alkyl, C₂-C₆ alkenyl, C₂-C₆ alkynyl, C₇-C₁₂ aralkyl, C₇-C₁₂ alkaryl, C₃-C₈ cycloalkyl, C₃-C₈ cycloalkenyl, phenyl, toluyl, xylenyl, biphenyl, C₁-C₆ alkoxy, C₁-C₆ alkenyloxy, C₁-C₆ alkynyloxy, C₂-C₁₂ alkoxyalkyl, C₂-C₁₂ alkoxyalkyloxy, C₂-C₁₂ alkylcarbonyl, C₂-C₁₂ alkylcarbonylamino, C₂-C₁₂ alkoxyamino, C₂-C₁₂ alkoxyaminocarbonyl, C₁-C₁₂ alkylamino, C₁-C₆ alkylthio, C₂-C₁₂ alkylthiocarbonyl, C₁-C₆ alkylsulfinyl, C₁-C₆ alkylsulfonyl, C₁-C₆ haloalkoxy, C₁-C₆ haloalkylsulfonyl, C₁-C₆ haloalkyl, C₁-C₆ hydroxyalkyl, -C(O)O(C₁-C₆ alkyl), - (CH₂)ₙ-O-(C₁-C₆ alkyl), benzyloxy, phenoxy, phenylthio, -(CONHSO₂R), -CHO, amino, amidino, bromo, carbamyl, carboxyl, carbalkoxy, -(CH₂)ₙ-CO₂H, chloro, cyano, cyanoguanidinyl, fluoro, guanidino, hydrazide, hydrazino, hydrazido, hydroxy, hydroxyamino, iodo, nitro, phosphono, -SO₃H, thioacetal, thiocarbonyl, and C₁-C₆ carbonyl; where n is from 1 to 8.

The term, "acidic group" means an organic group which when attached to an indene nucleus, through suitable linking atoms (hereinafter defined as the "acid linker"), acts as a proton donor capable of hydrogen bonding. Illustrative of an acidic group are the following: $\text{-5-tetrazolyl,} {\text{-SO}}_{\text{3}} \text{H,}$ 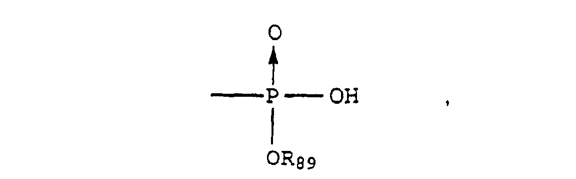 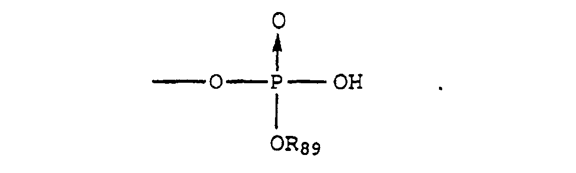 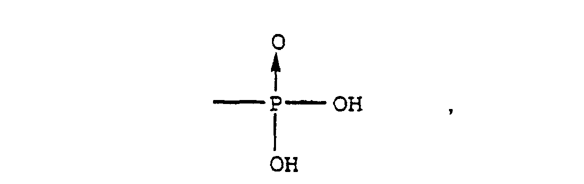 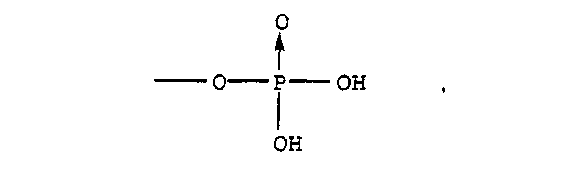 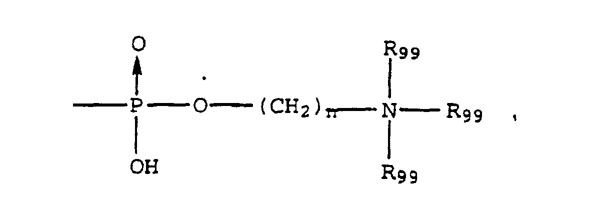 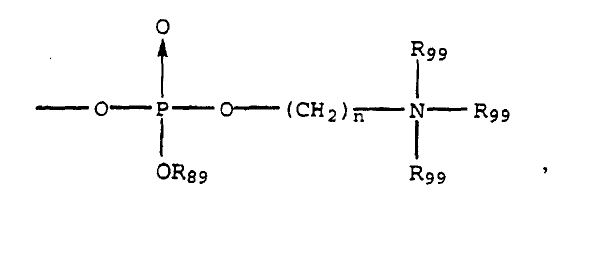 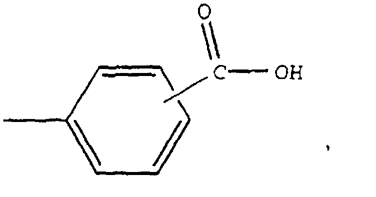 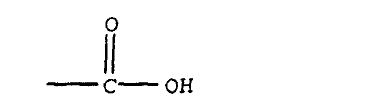 where n is 1 to 8, R₈₉ is a metal or C₁-C₁₀ alkyl, and R₉₉ is hydrogen or C₁-C₁₀ alkyl.

The words, "acid linker" refer to a divalent linking group symbolized as, -(Lₐ)-, which has the function of joining the indene nucleus to an acidic group in the general relationship:

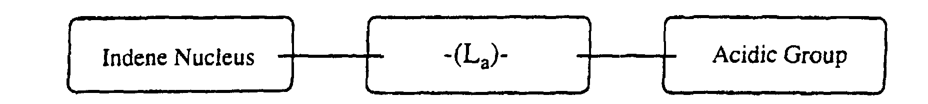

The words, "acid linker length", refer to the number of atoms (excluding hydrogen) in the shortest chain of the linking group -(Lₐ)- that connects the indene nucleus with the acidic group. The presence of a carbocyclic ring in -(Lₐ)- counts as the number of atoms approximately equivalent to the calculated diameter of the carbocyclic ring. Thus, a benzene or cyclohexane ring in the acid linker counts as 2 atoms in calculating the length of -(Lₐ)-. Illustrative acid linker groups are; 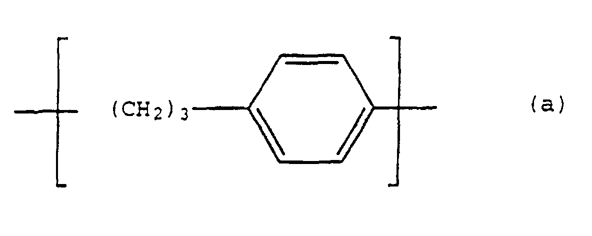 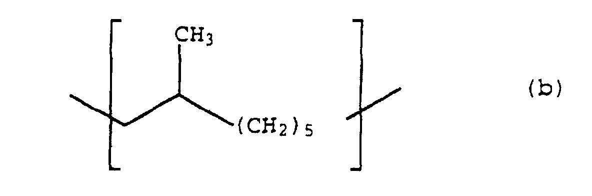 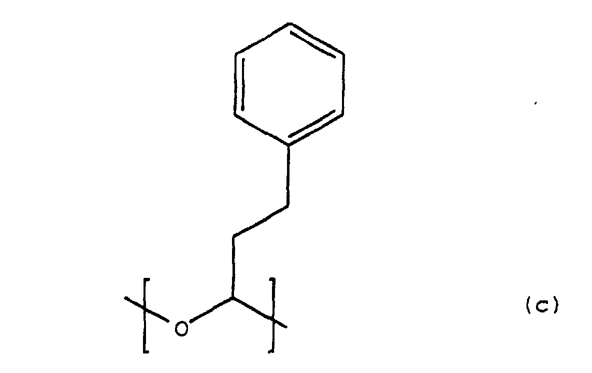 wherein, groups (a), (b), and (c) have acid linker lengths of 5, 7, and 2, respectively.

The term, "amine", includes primary, secondary and tertiary amines.

### The Indene Compounds :

There are three types of Indene compounds as represented by structural formulae (I), (II), and (III) below:
1) The indene-1-acetamides are represented by the formula (I),
   below: 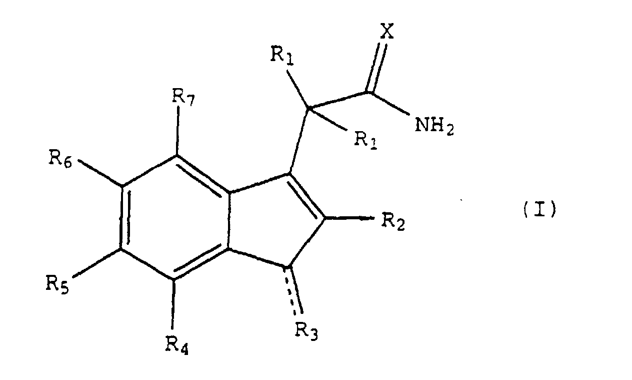 where X is oxygen or sulfur and each R₁ is independently hydrogen, C₁-C₃ alkyl, or halo and all other groups are as hereinafter defined.
2) The indene-1-hydrazides are represented by the formula (II), as set out below: 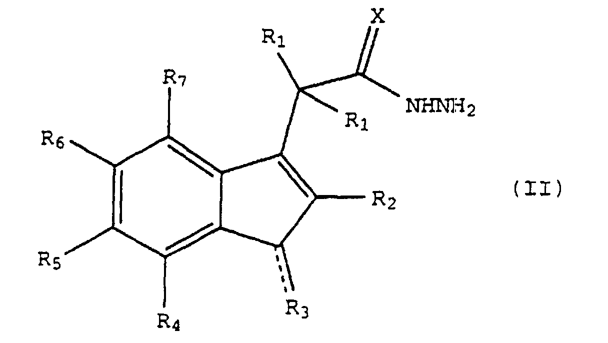 where X is oxygen or sulfur and each R₁ is independently hydrogen, C₁-C₃ alkyl, or halo and all other groups are as hereinafter defined.
3) The indene-1-glyoxylamides are represented by the formula (III), as set out below: 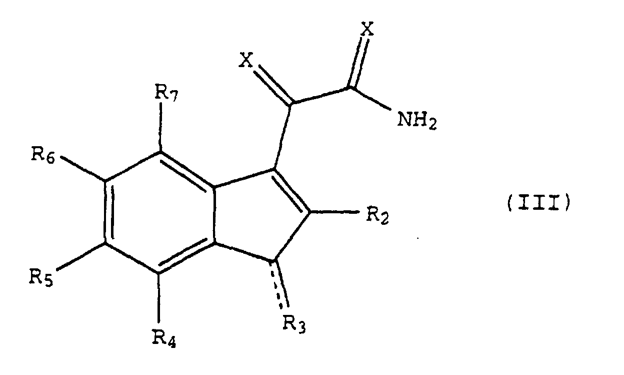
where X is independently oxygen or sulfur and all other groups are as hereinafter defined.

For formulae (I), (II), and (III) above the remaining groups are defined as follows:
R₃ is selected from groups (a), (b) and (c) where;
   (a) is C₇-C₂₀ alkyl, C₇-C₂₀ alkenyl, C₇-C₂₀ alkynyl, carbocyclic radical, or heterocyclic radical, or
   (b) is a member of (a) substituted with one or more independently selected non-interfering substituents; or
   (c) is the group -(L)-R₈₀; where, -(L)- is a divalent linking group of 1 to 12 atoms and where R₈₀ is a group selected from (a) or (b);
R₂ is hydrogen, halo, C₁-C₃ alkyl, C₃-C₄ cycloalkyl, C₃-C₄ cycloalkenyl, -O-(C₁-C₂ alkyl), -S-(C₁-C₂ alkyl), or a non-interfering substituent having a total of 1 to 3 atoms other than hydrogen; (that is, the R₁₂ radical may contain hydrogen atoms, but the remaining atoms comprising the total of 1 to 3 are non-hydrogen);
R₆ and R₇ are independently selected from hydrogen, a non-interfering substituent, or the group, -(Lₐ)-(acidic group); wherein -(Lₐ)-, is an acid linker having an acid linker length of 1 to 10; provided, that at least one of R₆ and R₇ must be the combined group, -(Lₐ)-(acidic group); and
R₄ and R₅ are each independently selected from hydrogen, non-interfering substituent, carbocyclic radical, carbocyclic radical substituted with non-interfering substituents, heterocyclic radical, and heterocyclic radical substituted with non-interfering substituents.

### Preferred Subgroups of Indene Compounds:

A preferred subclass of compounds of formulae (I), (II), and (III) are those wherein all X are oxygen.

Another preferred subclass of compounds of formulae (I), (II), and (III) are those wherein R₂ is selected from the group; halo, cyclopropyl, methyl, ethyl, -O-methyl, and -S-methyl.

Another preferred subclass of compounds of formulae (I), (II) and (III) are those wherein for R₃, -(L)- is selected from the group consisting of: 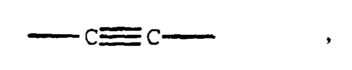 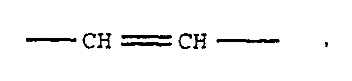 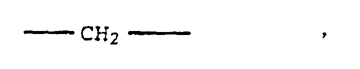 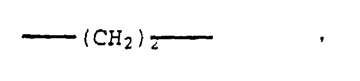 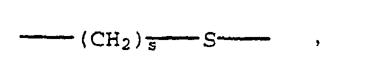 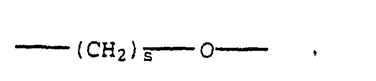 and 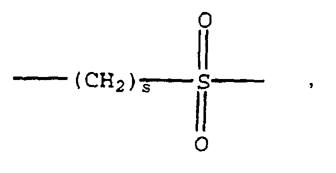 where s is 0 or 1.

Another preferred subclass of compounds of formulae (I), (II), and (III) are those wherein for R₃, group R₈₀ is carbocyclic, attached to the indene ring with a double or single bond, and is selected from the group consisting of benzylidene, cycloalkyl, cycloalkenyl, phenyl, naphthyl, norbornanyl, bicycloheptadienyl, toluyl, xylenyl, indenyl, stilbenyl, terphenylyl, diphenylethylenyl, phenyl-cyclohexenyl, acenaphthylenyl, and anthracenyl, biphenyl, bibenzylyl and related bibenzylyl homologues represented by the formula (bb), 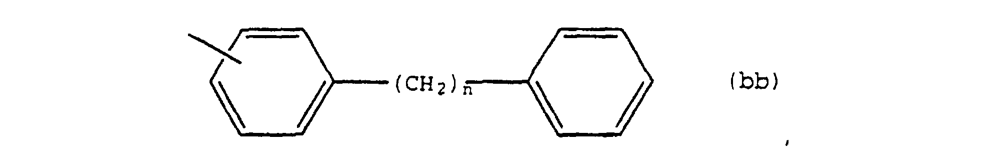 where n is a number from 1 to 8. Particularly preferred are compounds wherein R₃ is selected from the group consisting of 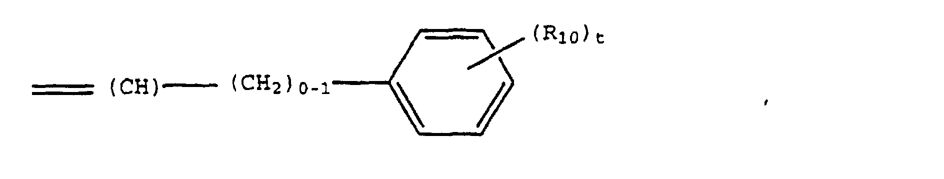 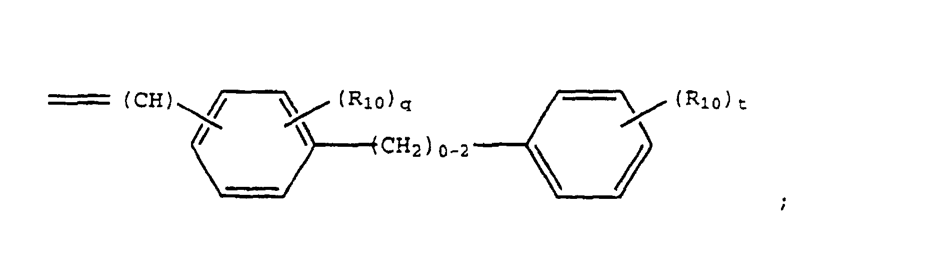 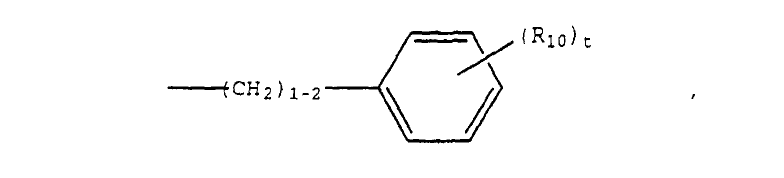 and 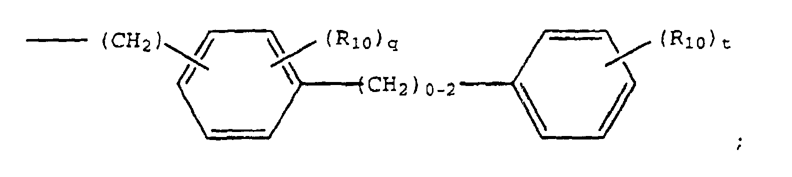 where R₁₀ is a radical independently selected from halo, C₁-C₁₀ alkyl, C₁-C₁₀ alkoxy, -S-(C₁-C₁₀ alkyl), and C₁-C₁₀ haloalkyl, q is a number from 0 to 4, and t is a number from 0 to 5.

Another preferred subclass of compounds of formulae (I), (II), and (III) are those wherein R₇ comprises an acidic group and the acid linker for the R₇ acidic group has an acid linker length of 2 or 3 and the acid linker group, -(Lₐ)-, for R₇ is selected from the group represented by the formula; 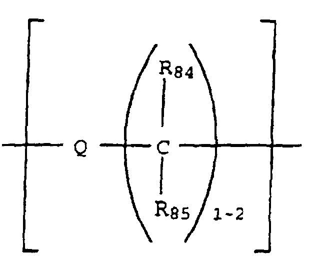 where Q is selected; from the group -(CH₂)-, -O-, -NH-, and -S-, and R₈₄ and R₈₅ are each independently selected from hydrogen, C₁-C₁₀ alkyl, aryl, C₁-C₁₀ alkaryl, C₁-C₁₀ aralkyl, carboxy, carbalkoxy, and halo. Most preferred are compounds where the acid linker, -(Lₐ)-, for R₇ is selected from the specific groups; 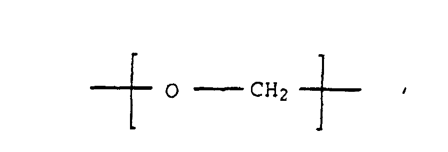 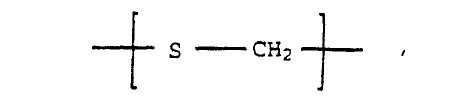 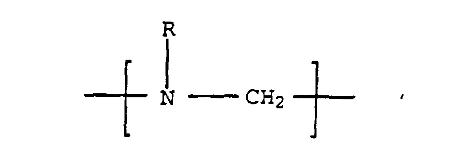 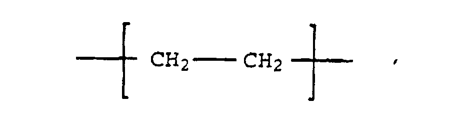 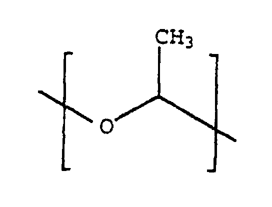 and 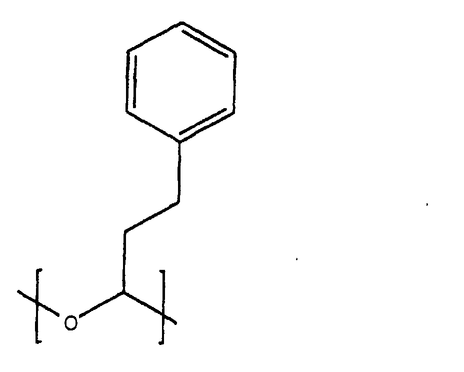 where R is H or C₁-C₄ alkyl.

Another preferred subclass of compounds of formulae (I), (II), and (III) are those wherein R₆ comprises an acidic group and the acid linker of the R₆ acidic group has an acid linker with an acid linker length of 3 to 10 atoms and the acid linker group, -(Lₐ)-, for R₆ is selected from; 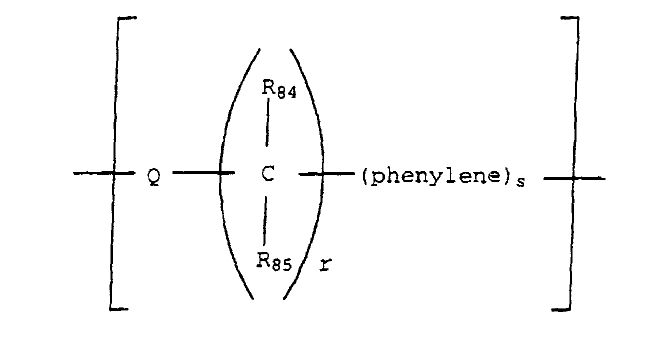 where r is a number from 1 to 7, s is 0 or 1, and Q is selected from the group -(CH₂)-, -O-, -NH-, and -S-, and R₈₄ and R₈₅ are each independently selected from hydrogen, C₁-C₁₀ alkyl, aryl, C₁-C₁₀ alkaryl, C₁-C₁₀ aralkyl, carboxy, carbalkoxy, and halo. Most preferred are compounds where the acid linker, -(Lₐ)-, for R₆ is selected from the specific groups; 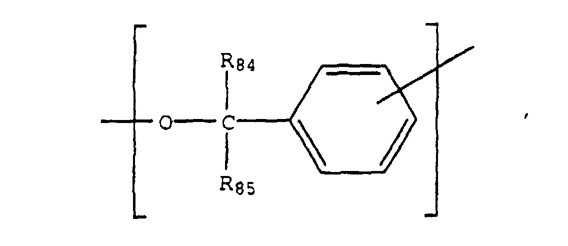 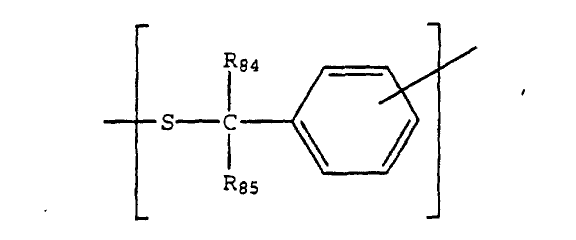 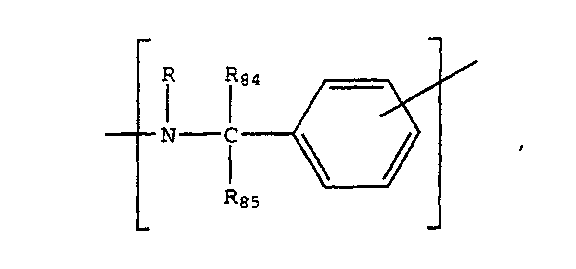 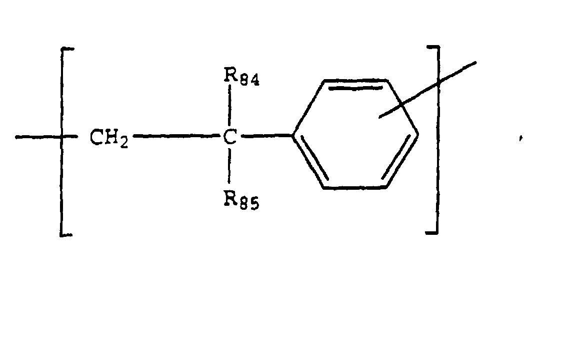 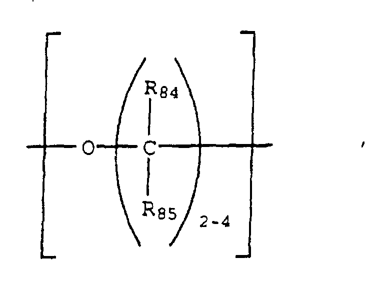 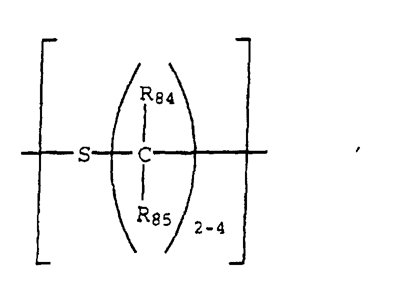 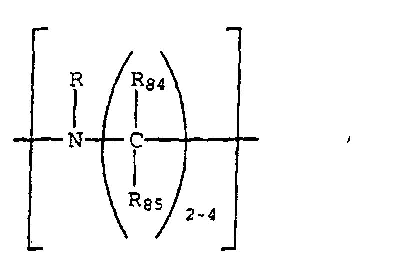 and 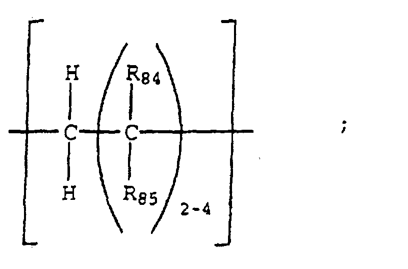 wherein; R is hydrogen or C₁-C₄ alkyl, R₈₄ and R₈₅ are each independently selected from hydrogen, C₁-C₁₀ alkyl, aryl, C₁-C₁₀ alkaryl, C₁-C₁₀ aralkyl, carboxy, carbalkoxy, and halo.

Another preferred subclass of compounds of formulae (I), (II), (III) are those wherein the acidic group (or salt, and prodrug derivatives thereof) on R₆ and/or R₇ is selected from the following: $\text{-5-tetrazolyl,} {\text{-SO}}_{\text{3}} \text{H,}$ 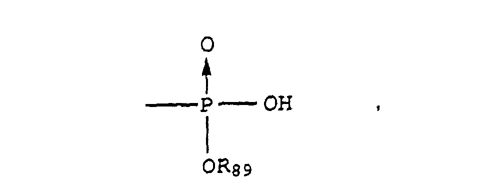 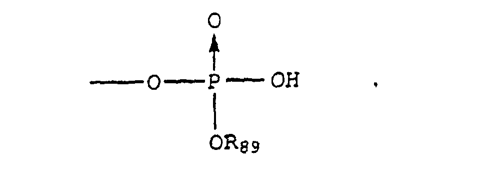 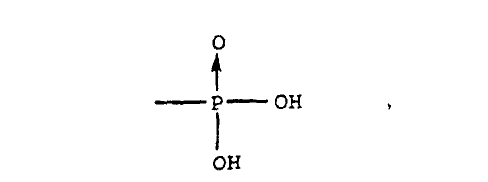 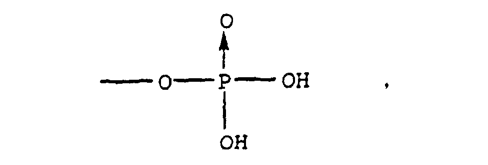 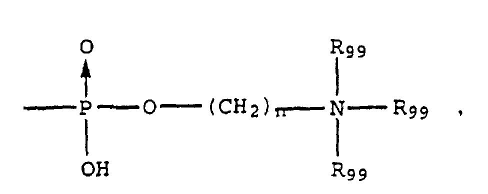 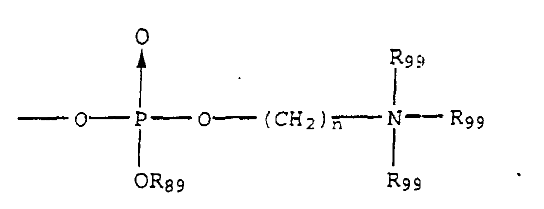 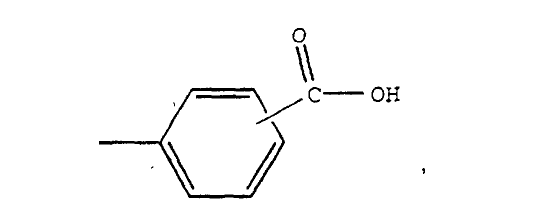 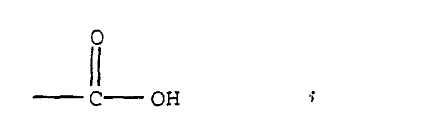 where n is 1 to 8, R₈₉ is a metal or C₁-C₁₀ alkyl, and R₉₉ is hydrogen or C₁-C₁₀ alkyl. Particularly preferred are compounds wherein the acidic group of R₆ and R₇ is selected from;

― CO₂H ,

― SO₃H,

― P(O)(OH)₂ ,

or salt, and prodrug (e.g., ester) derivatives thereof. The carboxyl group is the most preferred acidic group. It is highly preferred that only one of R₆ or R₇ contain an acidic group.

Another preferred subclass of compounds of formula (I), (II), and (III) are those wherein R₄ and R₅ are each independently selected from hydrogen and non-interfering substituents, with the non-interfering substituents being selected from the group consisting of C₁-C₆ alkyl, C₂-C₆ alkenyl, C₂-C₆ alkynyl, C₇-C₁₂ aralkyl, C₇-C₁₂ alkaryl, C₃-C₈ cycloalkyl, C₃-C₈ cycloalkenyl, phenyl, toluyl, xylenyl, biphenyl, C₁-C₆ alkoxy, C₁-C₆ alkenyloxy, C₁-C₆ alkynyloxy, C₂-C₁₂ alkoxyalkyl, C₂-C₁₂ alkoxyalkyloxy, C₂-C₁₂ alkylcarbonyl, C₂-C₁₂ alkylcarbonylamino, C₂-C₁₂ alkoxyamino, C₂-C₁₂ alkoxyaminocarbonyl, C₁-C₁₂ alkylamino, C₁-C₆ alkylthio, C₂-C₁₂ alkylthiocarbonyl, C₁-C₆ alkylsulfinyl, C₁-C₆ alkylsulfonyl, C₁-C₆ haloalkoxy, C₁-C₆ haloalkylsulfonyl, C₁-C₆ haloalkyl, C₁-C₆ hydroxyalkyl, -C(O)O(C₁-C₆ alkyl), -(CH₂)ₙ-O-(C₁-C₆ alkyl), benzyloxy, phenoxy, phenylthio, -(CONHSO₂R), -CHO, amino, amidino, bromo, carbamyl, carboxyl, carbalkoxy, -(CH₂)ₙ-CO₂H, chloro, cyano, cyanoguanidinyl, fluoro, guanidino, hydrazide, hydrazino, hydrazido, hydroxy, hydroxyamino, iodo, nitro, phosphono, -SO₃H, thioacetal, thiocarbonyl, and C₁-C₆ carbonyl; where n is from 1 to 8.

Specific preferred indene-1-acetamide type compounds of the invention (per formula I, supra) are represented by formulae 14a, 14b, 14c, 14d, 14e, 14f, 14g, 14h, 14i, 14j, 17a, 17b; and acceptable salts, solvates and prodrug derivatives thereof:

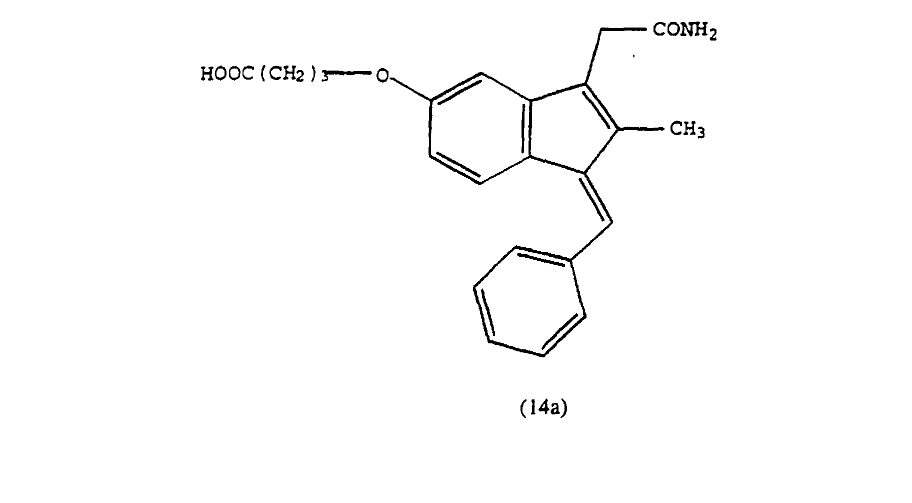

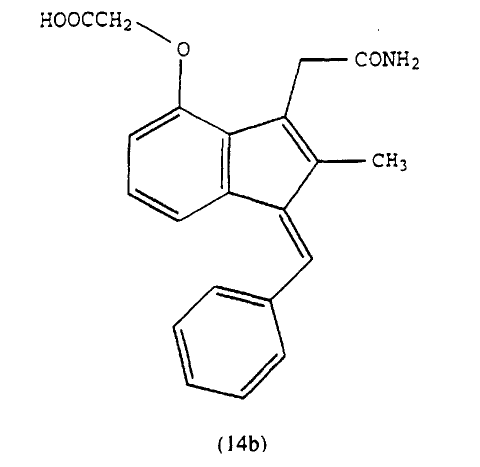

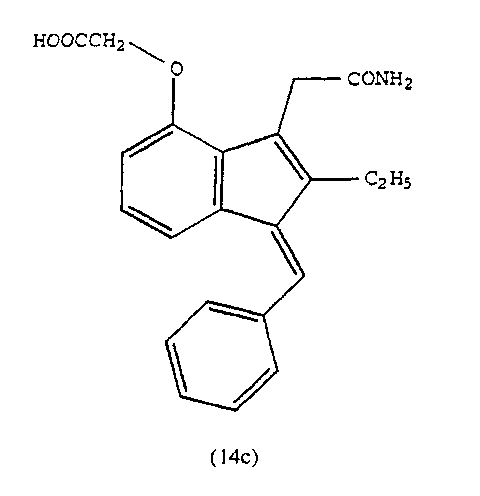

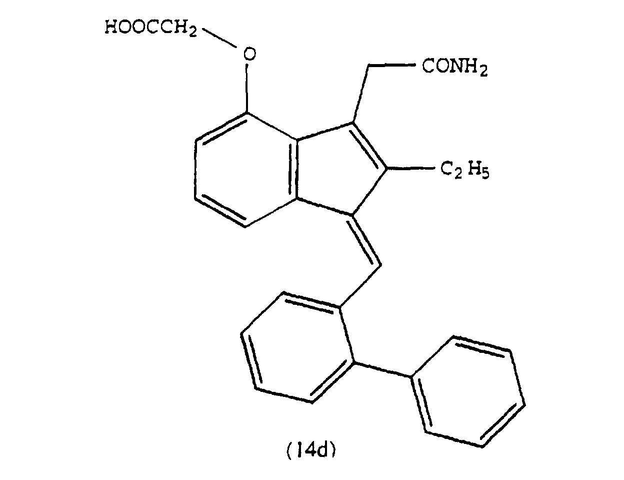

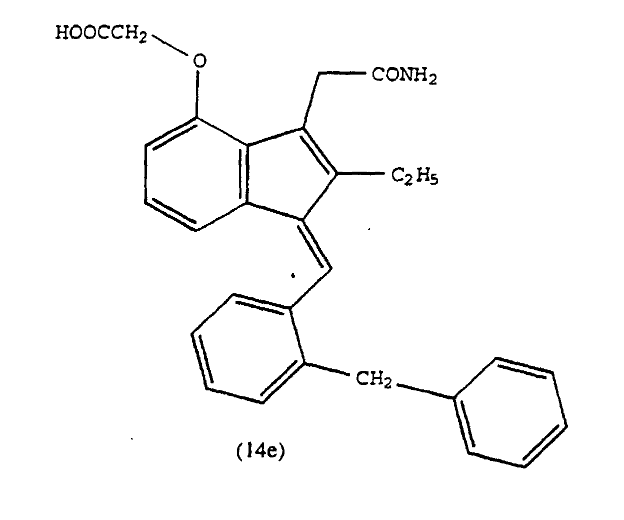

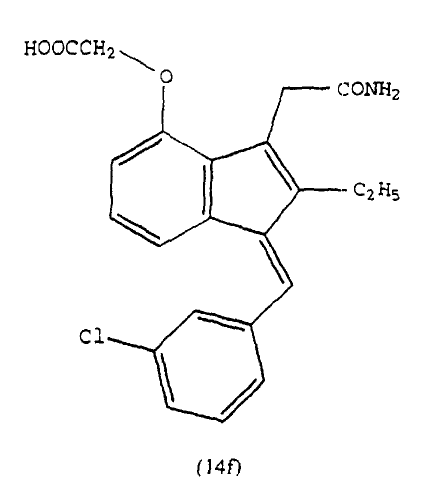

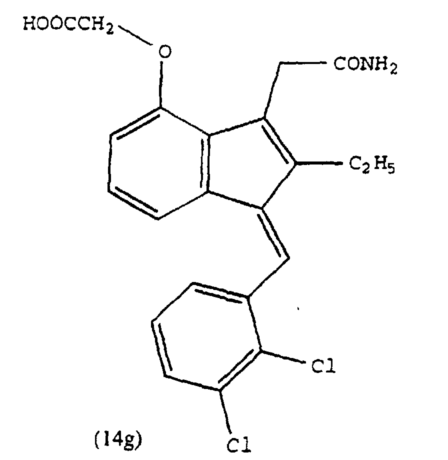

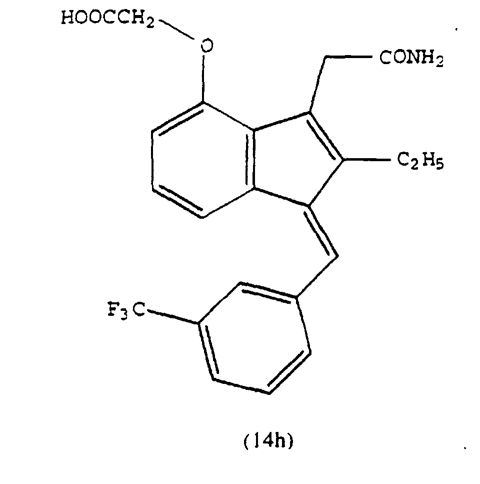

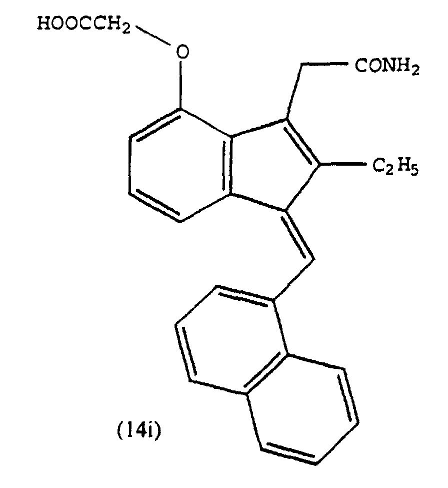

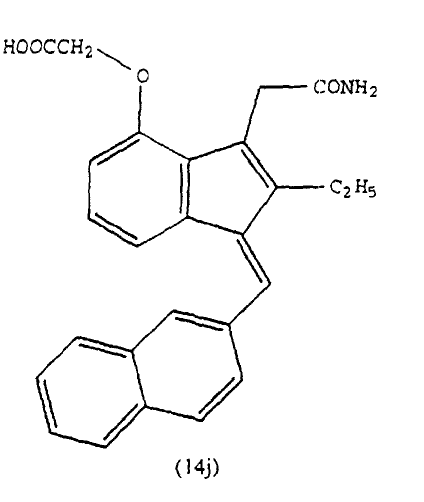

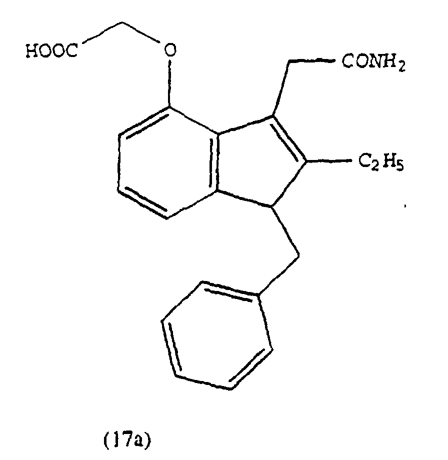

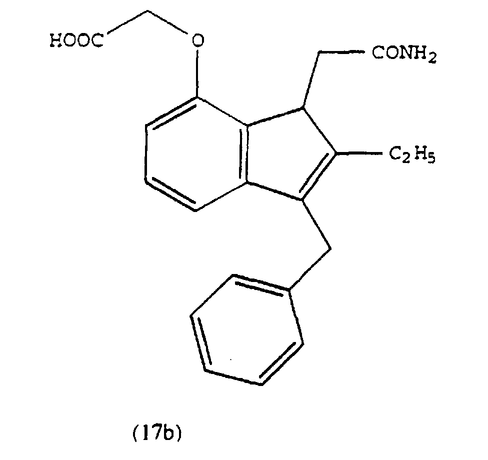

The salts of the above indene-1-functional compounds 14 a-j and 17a-b are an additional aspect of the invention. In those instances where the compounds of the invention possess acidic or basic functional groups various salts may be formed which are more water soluble and physiologically suitable than the parent compound. Representative pharmaceutically acceptable salts, include but are not limited to, the alkali and alkaline earth salts such as lithium, sodium, potassium, calcium, magnesium, aluminum and the like. Salts are conveniently prepared from the free acid by treating the acid in solution with a base or by exposing the acid to an ion exchange resin.

Included within the definition of pharmaceutically acceptable salts are the relatively non-toxic, inorganic and organic base addition salts of compounds of the present invention, for example, ammonium, quaternary ammonium, and amine cations, derived from nitrogenous bases of sufficient basicity to form salts with the compounds of this invention (see, for example, S. M. Berge, *et al.,* "Pharmaceutical Salts," J. Phar. Sci., 66: pp. 1-19 (1977)). Moreover, the basic group(s) of the compound of the invention may be reacted with suitable organic or inorganic acids to form salts such as acetate, benzenesulfonate, benzoate, bicarbonate, bisulfate, bitartrate, borate, bromide, camsylate, carbonate, chloride, clavulanate, citrate, chloride, edetate, edisylate, estolate, esylate, fluoride, fumarate, gluceptate, gluconate, glutamate, glycolylarsanilate, hexylresorcinate, bromide, chloride, hydroxynaphthoate, iodide, isothionate, lactate, lactobionate, laurate, malate, malseate, mandelate, mesylate methylbromide, methylnitrate, methylsulfate, mucate, napsylate, nitrate, oleate, oxalate, palmitate, pantothenate, phosphate, polygalacturonate, salicylate, stearate, subacetate, succinate, tannate, tartrate, tosylate, trifluoroacetate, trifluoromethane sulfonate, and valerate.

Certain compounds of the invention may possess one or more chiral centers and may thus exist in optically active forms. Likewise, when the compounds contain an alkenyl or alkenylene group there exists the possibility of cis- and trans- isomeric forms of the compounds. The R- and S-isomers and mixtures thereof, including racemic mixtures as well as mixtures of cis- and trans- isomers, are contemplated by this invention. Additional asymmetric carbon atoms can be present in a substituent group such as an alkyl group. All such isomers as well as the mixtures thereof are intended to be included in the invention. If a particular stereoisomer is desired, it can be prepared by methods well known in the art by using stereospecific reactions with starting materials which contain the asymmetric centers and are already resolved or, alternatively by methods which lead to mixtures of the stereoisomers and subsequent resolution by known methods.

Prodrugs are derivatives of the compounds of the invention which have chemically or metabolically cleavable groups and become by solvolysis or under physiological conditions the compounds of the invention which are pharmaceutically active in vivo. The prodrug derivative form often offers advantages of solubility, tissue compatibility, or delayed release in a mammalian organism (see, Bundgard, H., Design of Prodrugs, pp. 7-9, 21-24, Elsevier, Amsterdam 1985). Prodrugs include acid derivatives well known to practitioners of the art, such as, for example, esters prepared by reaction of the parent acidic compound with a suitable alcohol, or amides prepared by reaction of the parent acid compound with a suitable amine. Simple aliphatic or aromatic esters derived from acidic groups pendent on the compounds of this invention are preferred prodrugs. In some cases it is desirable to prepare double ester type prodrugs such as (acyloxy) alkyl esters or ((alkoxycarbonyl)oxy)alkyl esters.

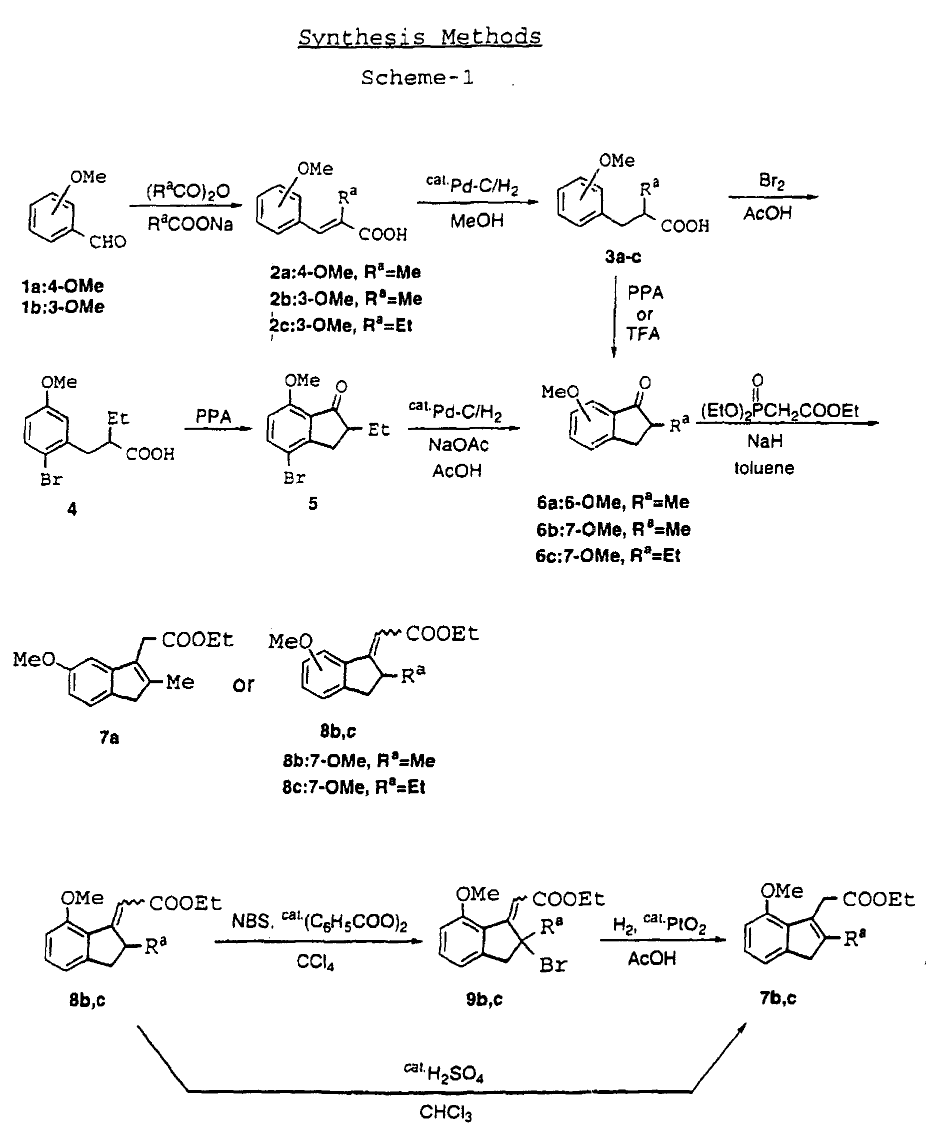

A mixture of an anisaldehyde 1, propionic anhydride, and sodium propionate is heated to produce 2 which is reduced by hydrogen in the presence of Pd/C to give 3. Acid cyclization of 3 yields 6. Alternatively, the aromatic position para to the methoxy group of 3 is blocked by bromination to give 4 which is cyclized to 5 by acid and then debrominated using hydrogen and Pd/C to give 6. Reaction of 6 with the anion of triethyl phosphonoacetate produces 7 and/or 8. Radical bromination of 8 gives 9, which on reduction with hydrogen in the presence of PtO₂ yields 7. Alternatively, treatment of 8 with acid gives 7.

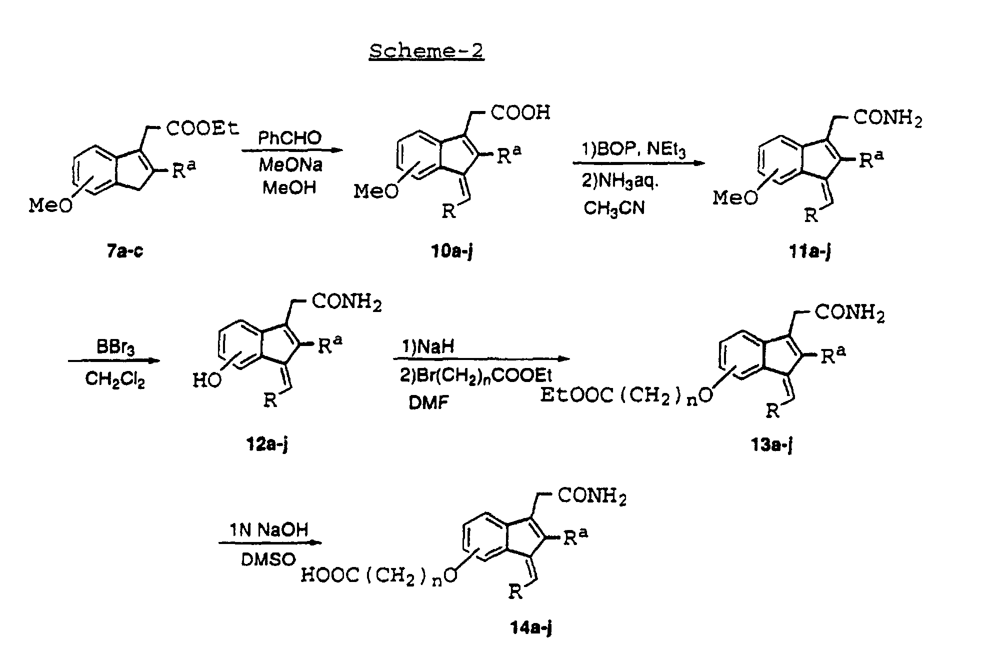

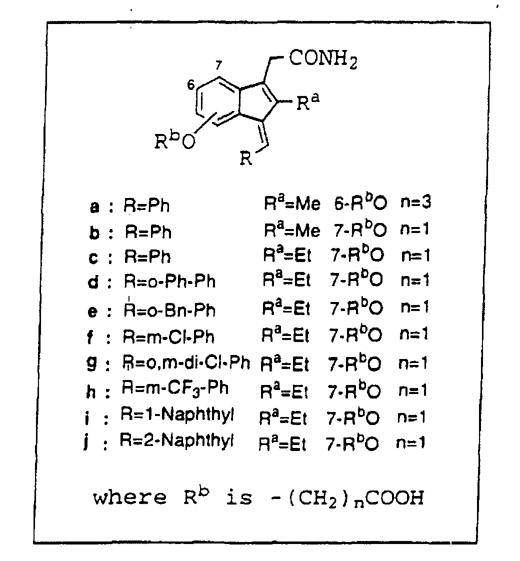

Compound 7 is condensed with benzaldehyde and its derivatives in the presence of base to give 10. Indenes 10 are converted to an active ester using benzotriazo-1-yloxytris(dimethylamino) hexafluorophosphonate and then reacted with ammonium hydroxide to form 11. Demethylation of 11 with BBr₃ forms 12 which is O-alkylated using sodium hydride and an omega-bromoalkanoic acid ester to produce 13. Aqueous base hydrolysis of 13 yields 14.

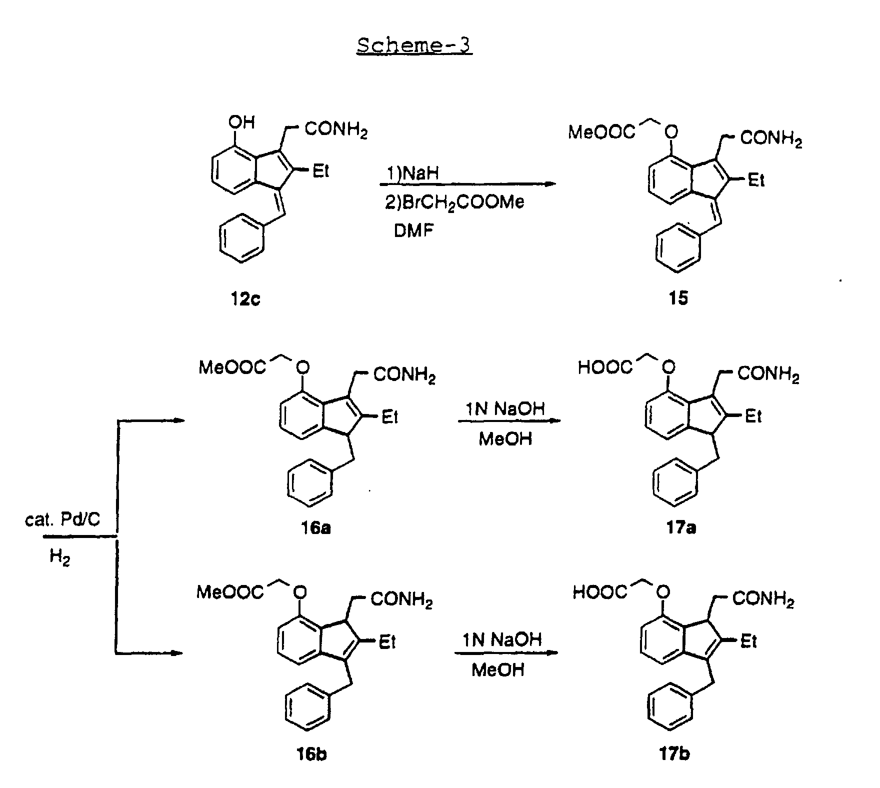

Compound 12c is O-alkylated using sodium hydride and methylbromoacetate to product 15 which is reduced by hydrogen in the presence of Pd/C to give a mixture of isomers 16a and 16b. Aqueous base hydrolysis of 16a and 16b gives 17a and 17b respectively.

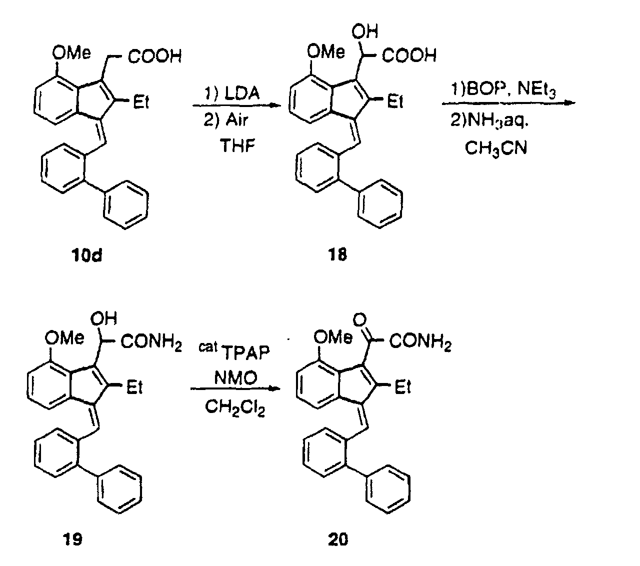

Compound 10d is treated with lithium diisopropylamine, then air is bubbled into the solution to give 18. The indene 18 is converted to an active ester using benzotriazo-1-yloxytris(dimethylamino)hexafluorophosphonate and then reacted with ammonium hydroxide to form the hydroxy acetamide 19. Compound 19 is oxidized to 20 using N-methylmorpholine N-oxide in the presence of tetrapropylammonium perruthenate.

### EXAMPLES

Reference numbers in the following Examples refer to compounds shown in the preceding Schemes.

### Example 1

This Example illustrates preparation of compounds 14a thru 14j (see, structural formulae, supra.) which are indene-3-acetamide type compounds of the invention.

### Part A: Preparation of 3-(4-Methoxyphenyl)-2-methylacrylic acid 2a

A mixture of p-anisaldehyde (26.6 g, 0.195 mol), propionic anhydride (43.0 ml, 0.347 mol) and sodium propionate (18.8 g, 0.195 mol) was heated at 150 °C overnight. After cooling, the reaction mixture was basified with 4N NaOH and washed with ether. The aqueous phase was acidified with concentrated HCl and the precipitate was filtered to yield 28.5 g (76%) of the titled compound, mp, 152-155 °C.
¹H NMR (CDCl₃) δ 2.16 (3H, d, J=1.2 Hz), 3.85 (3H, s), 6.95 (2H, d, J=8.7 Hz), 7.44 (2H, d, J=8.7 Hz), 7.78 (1H, br.s). IR νₘₐₓ (KBr) 1662, 1605, 1570, 1510 cm⁻¹. Analyses: Calc'd for C₁₁H₁₂O₃: C, 68.74; H, 6.29. Found: C, 68.49; H, 6.38. EIMS m/z=192 (M⁺, base peak).

Other Preparations:
3-(3-Methoxyphenyl)-2-methyl-acrylic acid 2b
Mp, 78-79 °C. 93% yield.
2-Ethyl-3-(3-methoxyphenyl)-acrylic acid 2c
Mp, 89-90 °C. 24% yield.

### Part B: Preparation of 3-(4-Methoxyphenyl)-2-methylpropionic acid 3a

A mixture of the acrylic acid (2a, 28.3 g, 0.147 mol) and 10% palladium-coal (2.49 g) in methanol (350 ml) was hydrogenated under 3 atm (3.0 x 10⁵ Pa.) overnight. The catalyst was filtered off and the solvent was evaporated to give the desired product (21.2 g, 74%). mp, 43-44 °C.

### Preparation of 3-(3-Methoxyphenyl)-2-methyl-propionic acid 3b

Using the procedure described in the synthesis of compound 3a from 2a, the acrylic acid (7, 38.2 g, 0.199 mol), was hydrogenated under atmosphere to give the desired product (29.0 g, 75%).

### Preparation of

2-(3-Methoxybenzyl)-butyric acid 3c. Quantitative yield.

### Part C: Preparation of 2-(2-Bromo-5-methoxy-benzyl)-butyric acid 4c

To a solution of the butyric acid (3a, 33.9 g, 0.163 m mol) in acetic acid (50 ml) was added a solution of bromine (10.5 ml, 0.204 m mol) in acetic acid (20 ml) at 0 °C. The resulting orange solution was stirred at room temperature for 2.5 hours and then the mixture was partitioned between water and AcOEt. The aqueous layer was extracted with AcOEt and the combined organic layers were washed with saturated sodium thiosulfate solution, water and brine, dried over MgSO₄, filtered and evaporated in vacuo. The residue (14, 51.8 g) was used to the next preparation without further purification, mp, 39-42 °C.

### Part D: Preparation of 4-Bromo-2-ethyl-7-methoxy-1-indanone 5c

A mixture of the butyric acid (4c, 51.6 g) and polyphosphoric acid (511 g) was heated at 100 °C for 2.5 hours. The mixture was poured into water (1 L) and extracted with AcOEt. The combined extracts were washed with water, 5% sodium bicarbonate solution and brine, dried over MgSO₄ and filtered. After removing the solvent under reduced pressure, the residue was chromatographed on silica gel eluting with hexane:AcOEt (4:1 to 1:2) to give the titled compound (30.0 g).

### Part E-1: Preparation of 6-Methoxy-2-methyl-1-indanone 6a

To the propionic acid (3a, 21.0 g, 0.108 mol) was added polyphosphoric acid (about 200 g) at 50 °C, the reaction was maintained at 90 °C for 2 hours. The mixture was poured into water (1 L), stirred at room temperature overnight and extracted with ether twice. The combined extracts were washed with saturated sodium bicarbonate solution and brine, dried over MgSO₄ and filtered. After removing the solvent under reduced pressure, the residue was chromatographed on silica gel eluting with CH₂Cl₂:MeOH (10:0 to 9:1) and hexane:AcOEt (9:1 to 4:1) to give the titled compound (7.45 g, 39%).
¹H NMR (CDCl₃) δ 1.31 (3H, d J=7.2 Hz), 2.58-2.84 (2H, m), 3.33 (1H, dd, J=16.1, 7.1 Hz), 3.84 (3H, s), 7.15-7.23 (2H, m), 7.30-7.40 (1H, m).

### Part E-2: Preparation of 7-Methoxy-2-methyl-1-indanone 6b

To a solution of the propionic acid (3b, 17.4 g, 0.0897 mol) in trifluoroacetic acid (300 ml) was slowly added trifluoroacetic anhydride (70 ml) at 0 °C and the reaction was maintained at this temperature for 40 min. After removing the solvent, the residue was dissolved with ether and poured into 10% NaOH. The organic layer was separated and washed with 10% NaOH, water and brine. The aqueous layer was extracted with ether and the combined extracts were dried over MgSO₄, filtered and concentrated in vacuo. The residue was chromatographed on silica gel eluting with hexane:AcOEt (9:1 to 1:1) to give the titled compound (2.66 g, 17%).

### Part E-3: Preparation of 2-Ethyl-7-methoxy-1-indanone 6c

A mixture of the indanone (5c, 29.7 g, 0.110 mol), sodium acetate (15.0 g, 0.183 mol), 10% palladium-coal (3.01 g) in acetic acid (300 ml) was hydrogenated at ordinary pressure. The catalyst was filtered off and the solvent was evaporated. The residue was partitioned between water and AcOEt and the aqueous layer was extracted with AcOEt. The combined organic layers were washed with water, 5% NaHCO₃ and brine, dried, filtered and evaporated. The crude product was purified by recrystallization from hexane:AcOEt to give the pure titled compound (15.7 g, 3 steps yield 51%), mp, 67-69 °C.

### Part F-1: Preparation of Ethyl (6-methoxy-2-methyl-3H-indene-1-yl)-acetate 7a

To a solution of triethyl phosphonoacetate (17.2 g, 76.7 m mol) in toluene (100 ml) was added sodium hydride (60% oil suspension, 3.06 g, 76.5 m mol) at 0 °C. After the reaction mixture was stirred for 1 hour at this temperature, a solution of the indanone (6a, 1.35 g, 7.63 m mol) in toluene (25 ml) was slowly added. The mixture was refluxed for 4 hours, then was poured into 1N HCl and extracted with AcOEt. The extract was washed with water and brine, dried over MgSO₄, filtered and concentrated to produce a crude residue, which was chromatographed on silica gel eluting with hexane:AcOEt (19:1) to give the mixture of the compound 7a and its positional isomer (1.65 g, 88%). The mixture was used to the next preparation without further purification.
¹H NMR (CDCl₃) δ 1.24 (3H, t, J=7.2 Hz), 2.12 (3H, s), 3.27 (2H, s), 3.49 (2H, s), 3.83 (3H, s), 4.14 (2H, q, J=7.2 Hz), 6.68 (1H, dd, J=2.5, 8.1 Hz), 6.86 (1H, d, J=2.5 Hz), 7.24 (1H, d, J=8.1 Hz).

### Part G: Preparation of Ethyl (7-methoxy-2-methyl-inden-1-ylidene)-acetate 8b

To a solution of triethyl phosphonoacetate (12.7 g, 56.7 m mol) in toluene (100 ml) was added sodium hydride (60% oil suspension, 2.29 g, 57.3 m mol) at 0 °C. After the reaction mixture was stirred for 70 min at this temperature, a solution of the indanone (6b, 2.00 g, 11.4 m mol) in toluene (28 ml) was added. The mixture was refluxed for overnight, then was poured into 2N HCl and extracted with AcOEt. The extract was washed with water and brine, dried over MgSO₄, filtered and concentrated to produce a crude residue, which was chromatographed on silica gel eluting with hexane:AcOEt (9:1) to give the titled compound (1.36 g, 49%).
¹H NMR (CDCl₃) δ 1.24 (3H, d, J=6.6 Hz), 1.27 (3H, t, J=7.0 Hz), 2.50-2.71 (1H, m), 2.93-3.19 (2H, m), 3.81 (3H, s), 4.21 (2H, q, J=7.0 Hz), 5.81 (1H, d, J=2.0 Hz), 6.70 (1H, d, J=8.4 Hz), 6.86 (1H, d, J=7.6 Hz), 7.25 (1H, t, J=7.8 Hz).

### Preparation of

Ethyl (2-ethyl-7-methoxyindan-1-yliden)-acetate 8c 36% yield.

### Part H-1: Preparation of Ethyl (2-bromo-7-methoxy-2-methyl-indan-1-ylidene)-acetate 9b

To a solution of the ester compound (8a, 1.39 g, 5.65 m mol) in carbon tetrachloride (15 ml) were added N-bromosuccinimide (1.11 g, 6.22 m mol) and benzoyl peroxide (68.5 mg, 0.283 m mol). The mixture was stirred at 50 °C for 6 hours, filtered, washed with saturated sodium thiosulfate solution and dried. After removing the solvent, the residue was chromatographed on silica gel eluting with hexane:AcOEt (19:1) to afford the titled compound (0.424 g, 23%), mp, 85-91 °C.
¹H NMR (CDCl₃) δ 1.23 (3H, t, J=7.2 Hz), 2.12 (3H, s), 3.42 (2H, s), 3.84 (3H, s), 4.22 (2H, qd, J=7.2, 1.0 Hz), 6.02 (1H, s), 6.79 (1H, d, J=8.0 Hz), 7.01 (1H, dd, J=7.2, 0.8 Hz), 7.13 (1H, t, J=7.7 Hz). IR νₘₐₓ (KBr) 3435, 3063, 2956, 2939, 2908, 2837, 1750, 1611, 1580 cm⁻¹. EIMS m/z=199 (base peak), 324 (M⁺). HR-EIMS Calc'd for C₁₅H₁₇BrO₃: 324.0361 Found: 324.0377.

### Part H-2: Preparation of Ethyl (2-bromo-2-ethyl-7-methoxy-indan-1-ylidene)-acetate 9c

Using the procedure described in the preparation of compound 9b from 8b, the ester compound (17, 2.46 g, 9.44 m 5 mol) in carbon tetrachloride (30 ml) was reacted with N-bromosuccinimide (2.03 g, 11.4 m mol) and benzoyl peroxide (0.116 g, 0.48 m mol) to give the crude product (1.67 g). This compound was used to the next step without purification.

### Part F-2: Preparation of Ethyl (7-methoxy-2-methyl-3H-inden-1-yl)-acetate 7b

The mixture of the ester compound (9b, 388 mg, 1.19 m mol) and platinum dioxide (38.4 mg) in acetic acid (4.0 ml) was hydrogenated under ordinary atmosphere overnight. The catalyst was filtered off and the solvent was evaporated. The residue was chromatographed on silica gel eluting with hexane:AcOEt (97:3 to 9:1) and crystallized from hexane:AcOEt to give 170 mg (59%) of the titled compound, mp, 76-79 °C.

### Part F-3: Preparation of Ethyl (2-ethyl-7-methoxy-3H-inden-1-yl)-acetate 7c

(Method A): Using the procedure described in the preparation of compound 7b from 9b, the ester compound (9c, 1.60 g) and platinum dioxide (0.160 g) in acetic acid (16.0 ml) was hydrogenated to give the titled compound (0.747 g, 2 Steps yield 32%.).

(Method B): To a solution of the ester compound (8c, 4.15 g, 0.0160 mol) in chloroform (40 ml) were added three drops of concentrated sulfuric acid. The resulting solution was refluxed for 40 min, dried over K₂CO₃ and filtered. After removing the solvent under reduced pressure, the residue was chromatographed on silica gel eluting with hexane:AcOEt (98:2 to 95:5) to give the titled compound (3.55 g, 85%).

### Part I-1: Preparation of [(3Z)-Benzylidene-6-methoxy-2-methyl-3H-inden-l-yl]-acetic acid 10a

To a solution of the crude indene (7a, 1.64 g, 6.66 m mol) in methanol (20 ml) were added benzaldehyde (1.35 ml, 13.3 m mol) and 1N NaOMe (20.0 ml, 20 m mol) at room temperature. The resulting solution was refluxed for 160 min, evaporated, poured into 1N HCl and extracted with AcOEt. The organic layer was washed with water and brine, dried over MgSO₄, filtered and concentrated in vacuo. The residue was subjected successively to chromatography on silica gel eluting with hexane:AcOEt (9:1) and then CHCl₃:MeOH (9:1) and to recrystallization (hexane:AcOEt) to afford 0.841 g (41% yield) of the desired product 10a, mp, 146-160 °C.
¹H NMR (CDCl₃) δ 2.19 (3H, s), 3.59 (2H, s), 3.78 (3H, s), 6.40 (1H, dd, J=8.2, 2.4 Hz), 6.75 (1H, d, J=2.4 Hz), 7.12 (1H, s), 7.24 (1H, d, J=8.2 Hz), 7.30-7.56 (5H, m). IR νₘₐₓ (KBr) 3428, 3007, 2935, 2834, 1701, 1611, 1582 cm⁻¹. Analyses: Calc'd for C₂₀H₁₈O₃ 0.2H₂O: C, 77.50; H, 5.98. Found: C, 77.35; H, 5.97. EIMS m/z=306 (M⁺, base peak).

Other Preparations:
[3(Z)-Benziliden-7-methoxy-2-methyl-3H-inden-1-yl]-acetic acid 10b. Mp, 165-175 °C. 59% yield.
[(3Z)-Benzylidene-2-ethyl-7-methoxy-3H-inden-1-yl]-acetic acid 10c. Decomp, 166-188 °C. 47% yield.
(Z)-2-[3-(Biphenyl-2-ylmethylene)-2-ethyl-7-methoxy-3H-inden-1-yl]-acetic acid 10d. Mp., 230-232 °C. 39% yield
(E/Z)-[3-(2-Benzyl-benzylidene)-2-ethyl-7-methoxy-3H-inden-1-yl]-acetic acid 10e. Mp, 119.5-136 °C. 62% yield.
(Z)-[3-(3-Chloro-benzylidene)-2-ethyl-7-methoxy-3H-inden-1-yl]-acetic acid 10f. Mp, 173-178 °C. 79% yield.
(Z)-[3-(2,3-Dichloro-benzylidene)-2-ethyl-7-methoxy-3H-inden-1-yl]-acetic acid 10g. Mp, 214.5-222 °C. 63% yield.
(Z)-(2-Ethyl-7-methoxy-3-naphthalen-1-ylmethylene-3H-inden-1-yl)-acetic acid 10i. Mp, 188-191 °C. 46% yield.
(Z)-(2-Ethyl-7-methoxy-3-naphthalen-2-ylmethylene-3H-inden-1-yl)-acetic acid 10j. Mp, 188-192.5 °C. 56% yield.

### Part J: Preparation of 2-[3(Z)-Benzylidene-6-methoxy-2-methyl-3H-inden-1-yl]-acetamide 11a

To a solution of the acetic acid derivative (10a, 200 mg, 0.645 m mol) in acetonitrile (10.0 ml) were added triethylamine (0.140 ml, 1.00 m mol) and benzotriazol-1-yloxytris(dimethylamino)phosphonium hexafuluorophoshate (434 mg, 0.981 m mol) at 0 °C. The resulting solution was stirred at room temperature for 45 min, and then 28% aqueous ammonia (0.440 ml) was added. After 30 min, the mixture was poured into 2N HCl and extracted with AcOEt. The organic layer was washed with water and brine, dried over MgSO₄ and filtered. After removing the solvent at reduced pressure, the residue was chromatographed twice on silica gel eluting with CH₂Cl₂:MeOH (99:1 to 98.5:1.5) and hexane:AcOEt (1:1 to 1:9) to give 183 mg (92%) of the titled compound, mp, 174-178 °C. ¹H NMR (CDCl₃) δ 2.22 (3H, s), 3.55 (2H, s), 3.79 (3H, s), 5.44 (1H, br.s), 5.64 (1H, br.s), 6.44 (1H, dd, J=8.4, 2.4 Hz), 6.73 (1H, d, J=2.4 Hz), 7.16 (1H, s), 7.29 (1H, d, J=8.4 Hz), 7.32-7.60 (5H, m). IR νₘₐₓ (KBr) 3389, 3196, 2913, 1653, 1614 cm⁻¹. Analyses: Calc'd for C₂₀H₁₉NO₂ 0.3H₂O: C, 77.30; H, 6.36; N, 4.51. Found: C, 77.18; H, 6.35; N, 4.40. EIMS m/z=305 (M⁺, base peak).

Other Preparations:
2-[3(Z)-Benzilidene-7-methoxy-2-methyl-3H-inden-1-yl]-acetamide 11b. Mp, 175-177 °C. 96% yield.
2-[(3Z)-Benzylidene-2-ethyl-7-methoxy-3H-inden-1-yl]-acetamide 11c. Mp, 153-156 °C. 99% yield.
(Z)-2-[3-(Biphenyl-2-ylmethylene)-2-ethyl-7-methoxy-3H-inden-1-yl]-acetamide 11d. Mp., 190-200 °C. 91% yield.
(E/Z)-2-[3-(2-Benzyl-benzylidene)-2-ethyl-7-methoxy-3H-inden-1-yl]-acetamide 11e. Mp, 133-147 °C. 74% yield.
(Z)-2-[3-(3-Chloro-benzylidene)-2-ethyl-7-methoxy-3H-inden-1-yl]-acetamide 11f. Mp, 204-208 °C. 85% yield.
(Z)-2-[3-(2,3-Dichloro-benzylidene)-2-ethyl-7-methoxy-3H-inden-1-yl]-acetamide 11g. Mp, 170-176.5 °C. 81% yield.
(Z)-2-[2-Ethyl-7-methoxy-3-(3-trifluoromethyl-benzylidene)-3H-inden-1-yl]-acetamide 11h. Mp, 205.5-208 °C. 32% yield (2 steps).
(Z)-2-(2-Ethyl-7-methoxy-3-naphthalen-1-ylmethylene-3H-inden-1-yl)-acetamide 11i. Mp, 172-175.5 °C. 91% yield.
(Z)-2-(2-Ethyl-7-methoxy-3-naphthalen-2-ylmethylene-3H-inden-1-yl)-acetamide 11j. Mp, 221-223 °C. 80% yield.

### Part K: Preparation of 2-[3(Z)-Benzylidene-6-hydroxy-2-methyl-3H-inden-l-yl]-acetamide 12a

To a solution of the acetamide (11a, 50.9 mg, 0.167 m mol) in dichloromethane (2.0 ml) was added dropwise borontribromide (1M in CH₂Cl₂ solution, 0.860 ml, 0.860 m mol) at 0°C and the resulting mixture was stirred at room temperature for 80 min. After evaporation, methanol (2.0 ml) was added. The solution was stirred and evaporated The residue was partitioned between 2N HCl and AcOEt, the extract was washed with water and brine, dried over MgSO₄ and concentrated in vacuo. The crude product was purified by preparative TLC on silica gel (200x200x0.25mm, elution with AcOEt) and recrystallization from hexane:AcOEt gave the titled compound (23.7 mg, 49%), mp, 178-180 °C.
¹H NMR (CDCl₃) δ 2.20 (3H, s), 3.56 (2H, s), 5.55 (1H, br.s), 5.77 (1H, br.s), 6.41 (1H, d, J=8.4, 2.4 Hz), 6.69 (1H, d, J=2.4 Hz), 7.14 (1H, s), 7.25 (1H, d, J=8.4 Hz), 7.30-7.56 (6H, m). IR νₘₐₓ (KBr) 3416, 3212, 1657, 1602 cm⁻¹. Analyses: Calc'd for C₁₉H₁₇NO₂ 0.4H₂O: C, 76.44; H, 6.01; N, 4.69. Found: C, 76.66; H, 5.91; N, 4.63. EIMS m/z=232 (base peak), 291 (M⁺).

Other Preparations:
2-[3(Z)-Benzilidene-7-hydroxy-2-methyl-3H-inden-1-yl]-acetamide 12b
Mp, 200-203 °C. Quantitative yield.
(Z)-2-(3-Benzylidene-2-ethyl-7-hydroxy-3H-inden-1-yl)-acetamide 12c. Dec.p., 176-180 °C. 87% yield.
(Z)-2-[3-(Biphenyl-2-ylmethylene)-2-ethyl-7-hydroxy-3H-inden-1-yl]-acetamide 12d. Mp., 168-172 °C. 49% yield.
(Z)-2-[3-(2-Benzyl-benzylidene)-2-ethyl-7-hydroxy-3H-inden-1-yl]-acetamide 12e. Mp, 137.5-153.5 °C. 72% yield.
(Z)-2-[3-(3-Chloro-benzylidene)-2-ethyl-7-hydroxy-3H-inden-1-yl]-acetamide 12f. Mp, 185-185.5 °C. 79% yield.
(Z)-2-[3-(2,3-Dichloro-benzylidene)-2-ethyl-7-hydroxy-3H-inden-1-yl]-acetamide 12g. Mp, 193-194.5 °C. 90% yield.
(Z)-2-[2-Ethyl-7-hydroxy-3-(3-trifluoromethyl-benzylidene)-3H-inden-1-yl]-acetamide 12h. Mp, 184.5-186 °C. 96% yield.
(Z)-2-(2-Ethyl-7-hydroxy-3-naphthalen-1-ylmethylene-3H-inden-1-yl)-acetamide 12i. Mp, 189-189.5 °C. 92% yield.
(Z)-2-(2-Ethyl-7-hydroxy-3-naphthalen-2-ylmethylene-3H-inden-1-yl)-acetamide 12j. Mp, 200-201.5 °C. 65% yield.

### Part L-1: Preparation of Ethyl 4-[1(Z)-Benzylidene-3-carbamoylmethyl-2-methyl-1H-inden-5-yloxy]-butanate 13a

To a solution of the hydroxy compound (12a, 43.7 mg, 0.150 m mol) in dimethylformamide (1.0 ml) was added sodium hydride (60% oil suspension, 21.3 mg, 0.533 m mol). After addition, the mixture was stirred at room temperature for 60 min, and then ethyl 4-bromobutyrate (75 µl, 0.524 m mol) was added. The mixture was stirred at 0 °C for 45 min and then at room temperature for 75 min, and partitioned between 1N HCl and AcOEt. The organic layer was separated, washed with water and brine, dried over MgSO₄, filtered and concentrated in vacuo. The residue was purified by preparative TLC (200x200x0.25 mm, elution with AcOEt) to give the product (38.0 mg, 63%), mp, 124-128 °C.
¹H NMR (CDCl₃) δ 1.25 (3H, t, J=7.0 Hz), 1.99-2.17 (2H, m), 2.21 (3H, s), 2.50 (2H, t, J=7.2 Hz), 3.54 (2H, s), 3.98 (2H, t, J=6.3 Hz), 4.14 (2H, q, J=7.0 Hz), 5.50 (1H, br.s), 5.67 (1H, br.s), 6.42 (1H, dd, J=8.2, 2.4 Hz), 6.73 (1H, d, J=2.4 Hz), 7.16 (1H, s), 7.27 (1H, d, J=8.2 Hz), 7.32-7.58 (5H, m). IR νₘₐₓ (KBr) 3445, 2972, 2919, 1739, 1687,1655, 1614 cm⁻¹. Analyses: Calc'd for C₂₅H₂₇NO₄ 0.9H₂O: C, 71.20; H, 6.88; N, 3.32 Found: C, 71.13; H, 6.76; N, 3.52. EIMS m/z=115 (base peak), 405 (M⁺).

### Part L-2: Preparation of Ethyl [1(Z)-benzylidene-3-carbamoylmethyl-2-methyl-1H-inden-4-yloxy]-acetate 13b

Using the procedure described in the synthesis of compound 13a from 12a, the hydroxy compound (12b, 53.1 mg, 0.182 m mol) in dimethylformamide (1.0 ml) was reacted with sodium hydride (60% oil suspension, 26.1 mg, 0.653 m mol) and ethyl bromoacetate (72 µl, 0.649 m mol). Purification by preparative TLC (200x200x0.25 mm, elution with CHCl₃:MeOH=19:1) gave the product (53.1 mg, 77%), mp, 162-165 °C.

Other Preparations:
(Z)-Ethyl (3-benzylidene-1-carbamoylmethyl-2-ethyl-3H-inden-7-yloxy)-acetate 13c. Mp., 139-142 °C. 61% yield.
(Z)-Ethyl [3- (biphenyl-2-ylmethylene)-1-carbamoylmethyl-2-ethyl-3H-inden-7-yloxy]-acetate 13d. Mp., 161-163 °C. 76% yield.
Ethyl (Z)-[1-(2-benzyl-benzylidene)-3-carbamoylmethyl-2-ethyl-1H-inden-4-yloxy]-acetate 13e. Mp, 157.5-158.5 °C. 83% yield.
Ethyl (Z)-[3-carbamoylmethyl-l-(3-chloro-benzylidene)-2-ethyl-1H-inden-4-yloxy]-acetate 13f. Mp, 182.5-184 °C. 57% yield.
Ethyl (Z)-[3-carbamoylmethyl-1-(2,3-dichloro-benzylidene)-2-ethyl-1H-inden-4-yloxy]-acetate 13g. Mp, 185.5-187.5 °C. 83% yield.
Ethyl (Z)-[3-carbamoylmethyl-2-ethyl-1-(3-trifluoromethylbenzylidene)-1H-inden-4-yloxy]-acetate 13h. Mp, 175.5-182.5 °C. 76% yield.
Ethyl (Z)-(3-carbamoylmethyl-2-ethyl-1-naphthalen-1-ylmethylene-1H-inden-4-yloxy)-acetate 13i. Mp, 153.5-160 °C. 64% yield.
Ethyl (Z)-(3-carbamoylmethyl-2-ethyl-1-naphthalen-2-ylmethylene-1H-inden-4-yloxy)-acetate 13j. Mp, 201-204 °C. 77% yield.

### Part M-1: Preparation of 4-[1(Z)-Benzylidene-3-carbamoylmethyl-2-methyl-1H-inden-5-yloxy]-butanoic acid 14a

1N Sodium hydroxide (0.200 ml, 0.200 m mol) was added to a solution of the ester (13a, 39.4 mg, 0.0972 m mol) in dimethylsulfoxide (1.0 ml) at room temperature. The solution was stirred at room temperature for 75 min, partitioned between 1N HCl and AcOEt. The organic layer was separated, washed with water and brine, dried over MgSO₄, filtered and evaporated in vacuo. Recrystallization from AcOEt afforded the titled compound (19.5 mg, 53%), mp, 187-188 °C.
¹H NMR (d₆-DMSO) δ 1.82-2.02 (2H, m), 2.14 (3H, s), 2.36 (2H, t, J=7.0 Hz), 3.94 (1H, t, J=6.3 Hz), 6.42 (1H, dd, J=8.4, 2.0 Hz), 6.85 (1H, d, J=2.0 Hz), 6.96 (1H, br.s), 7.13 (1H, d, J=8.4 Hz), 7.16 (1H, s), 7.32-7.57 (6H, m). IR νₘₐₓ (KBr) 3462, 3342, 3190, 2936, 1715, 1697, 1647, 1613, 1582 cm⁻¹. Analyses: Calc'd for C₂₃H₂₃NO₄ 0.5H₂O: C, 71.49; H, 6.26; N, 3.62. Found: C, 71.47; H, 6.25; N, 3.47. EIMS m/z=232 (base peak), 377 (M⁺).

Other Preparations:
[1(Z)-Benzylidene-3-carbamoylmethyl-2-methyl-1H-inden-4-yloxy]-acetic acid 14b. Mp, 224-226 °C. 55% yield. ¹H NMR (d₆-DMSO) δ 2.12 (3H, s), 3.64 (2H, s), 4.69 (2H, s), 6.70-6.95 (4H, m), 7.19 (1H, br.s), 7.31 (1H, s), 7.35-7.57 (5H, m), 13.2 (1H, br.s). EIMS m/z=349 (base peak, M⁺). HR-EIMS Calc'd for C₂₁H₁₉NO₄: 349.1312, Found: 349.1311.
(Z)-(3-Benzylidene-1-carbamoylmethyl-2-ethyl-3H-inden-7-yloxy)-acetic acid 14c. Mp., 119-205 °C. 91% yield.
   ¹H NMR (300 MHz, d₆-DMSO) δ 1.15 (3H, t, J=7.5 Hz), 2.60 (2H, q, J=7.5 Hz), 3.63 (2H, s), 4.65 (2H, s), 6.72-6.94 (1H, br m), 6.76 (1H, t, J=7.5 Hz), 6.82 (1H, d, J=8.4 Hz), 6.90 (1H, d, J=7.2 Hz), 7.32 (1H, s), 7.30-7.58 (6H, m). IR νₘₐₓ (KBr) 3387, 3081, 3055, 3024, 2966, 2935, 2873, 1706, 1655, 1592, 1573, 1478, 1438, 1257, 1103, cm⁻¹. EIMS m/z=304 (base peak), 363 (M⁺). HR-EIMS Calc'd for C₂₂H₂₁NO₄: 363.1469, Found: 363.1485
(Z)-[1-(Biphenyl-2-ylmethylene)-3-carbamoylmethyl-2-ethyl-1H-inden-4-yloxy]-acetic acid 14d. Mp., 205-209 °C. 93% yield. ¹H NMR (300 MHz, d₆-DMSO) δ 0.91 (3H, t, J=7.5 Hz), 2.41 (2H, q, J=7.5 Hz), 3.59 (2H, s), 4.68 (2H, s), 6.72-6.90 (4H, m), 7.00 (1H, s), 7.26 (1H, br.s), 7.30-7.62 (9H, m). IR νₘₐₓ (KBr) 3403, 2959, 2931, 2869, 1719, 1689, 1660, 1600, 1577, 1478, 1437, 1258, 1231, 1096, 735, 700 cm⁻¹. Analyses: Calc'd for C₂₈H₂₅NO₄ 0.3H₂O: C, 76.18; H, 6.31; N, 2.96. Found: C, 76.14; H; 6.35; N, 2.99. EIMS m/z=165 (base peak), 439 (M⁺).
(Z)-[1-(2-Benzyl-benzylidene)-3-carbamoylmethyl-2-ethyl-1H-inden-4-yloxy]-acetic acid 14e. Mp, 194-200 °C. Quantitative yield. ¹H NMR (d₆-DMSO) δ 1.07 (3H, t, J=7.4 Hz), 2.36 (2H, q, J=7.4 Hz), 3.61 (2H, s), 3.97 (2H, s), 4.68 (2H, s), 6.48 (1H, dd, J=6.4, 2.0 Hz), 6.67-6.80 (2H, m), 6.80-6.91 (1H, br), 7.04-7.43 (11H, m). IR νₘₐₓ (KBr) 3414, 1725, 1647, 1597, 1479, 1438 cm⁻¹. EIMS m/z=179 (base peak), 453 (M⁺). HR-EIMS Calc'd for C₂₉H₂₇ NO₄: 453.1938, Found: 453.1944.
(Z)-[3-Carbamoylmethyl-1-(3-chloro-benzylidene)-2-ethyl-1H-inden-4-yloxy]-acetic acid 14f. Mp, 213.5-214 °C. 82% yield. ¹H NMR (d₆-DMSO) δ 1.14 (3H, t, J=7.4 Hz), 2.58 (2H, q, J=7.4 Hz), 3.62 (2H, s), 4.69 (2H, s), 6.73-6.94 (4H, m), 7.23-7.28 (1H, br), 7.28 (1H, s), 7.43-7.62 (4H, m). IR νₘₐₓ (KBr) 3458, 3334, 2966, 1734, 1634, 1592, 1478, 1435 cm⁻¹. Analyses: Calc'd for C₂₂H₂₀ NO₄Cl 0.3H₂O: C, 65.53; H, 5.15; N, 3.47; Cl, 8.79. Found: C, 65.57; H, 5.19; N, 3.58; Cl, 8.83. EIMS m/z=338 (base peak), 397 (M⁺).
(Z)-[3-Carbamoylmethyl-1-(2,3-dichloro-benzylidene)-2-ethyl-1H-inden-4-yloxy]-acetic acid 14g. Mp, 226-228.5 °C. 89% yield. ¹H NMR (d₆-DMSO) δ 1.17 (3H, t, J=7.4 Hz), 2.60 (2H, q, J=7.4 Hz), 3.63 (2H, s), 4.68 (2H, s), 6.48 (1H, dd,
J=6.4, 1.6 Hz), 6.73-6.94 (3H, m), 7.17 (1H, s), 7.22-7.38 (1H, br), 7.38-7.61 (2H, m), 7.73 (1H, dd, J=7.8, 1.6 Hz). IR νₘₐₓ (KBr) 3415, 2966, 1726, 1650, 1600, 1479, 1435, 1409 cm⁻¹. EIMS m/z=378 (base peak), 431 (M⁺). HR-EIMS Calc'd for C₂₂H₁₉ NO₄Cl₂: 431.0689, Found: 431.0685.
(Z)-[3-Carbamoylmethyl-2-ethyl-1-(3-trifluoromethylbenzylidene)-1H-inden-4-yloxy]-acetic acid 14h. Mp, 207-211.5 °C. 70% yield. ¹H NMR (d₆-DMSO) δ 1.16 (3H, t, J=7.4 Hz), 2.60 (2H, q, J=7.4 Hz), 3.63 (2H, s), 4.69 (2H, s), 6.70-6.93 (4H, m), 7.12-7.30 (1H, br), 7.36 (1H, s), 7.64-7.91 (4H, m). IR νₘₐₓ (KBr) 3428, 1730, 1646, 1587, 1438, 1328 cm⁻¹. Analyses: Calc'd for C₂₃H₂₀ NO₄F₃: C, 64.04; H, 4.67; N, 3.25; F, 13.21. Found: C, 63.76; H, 4.71; N, 3.29; F, 13.02. EIMS m/z=372 (base peak), 431 (M⁺).
(Z)-(3-Carbamoylmethyl-2-ethyl-1-naphth-1-ylmethylene-1H-inden-4-yloxy)-acetic acid 14i. Mp, 210.5-213 °C. 90% yield. ¹H NMR (d₆-DMSO) δ 1.26 (3H, t, J=7.4 Hz), 2.72 (2H, q, J=7.4 Hz), 3.66 (2H, s), 4.66 (2H, s), 6.29 (1H, d, J=6.6 Hz), 6.56-6.74 (2H, m), 6.80-6.94 (1H, br), 7.17-7.35 (1H, br), 7.46-7.74 (5H, m), 7.86-8.10 (3H, m). IR νₘₐₓ (KBr) 3415, 2961, 1662, 1599, 1478, 1430, 1340 cm⁻¹. EIMS m/z=354 (base peak), 413 (M⁺). HR-EIMS Calc'd for C₂₆H₂₃NO₄: 413.1624, Found: 413.1621.
(Z)-(3-Carbamoylmethyl-2-ethyl-1-naphth-2-ylmethylene-1H-inden-4-yloxy)-acetic acid 14j. Mp, 210.5-215.5 °C. 70% yield. ¹H NMR (CDCl₃) δ 1.27 (3H, t, J=7.6 Hz), 2.75 (2H, q, J=7.6 Hz), 3.80 (2H, s), 4.68 (2H, s), 6.65 (1H, d, J=8.0 Hz), 6.80 (1H, t, J=8.0 Hz), 7.09 (1H, d, J=8.0 Hz), 7.39-7.67 (6H, m), 7.77-8.04 (4H, m). IR νₘₐₓ (KBr) 3434, 2965, 1716, 1662, 1478, 1430 cm⁻¹. EIMS m/z=354 (base peak), 413 (M⁺). HR-EIMS Calc'd for C₂₆H₂₃NO₄: 413.1626, Found: 413.1629.

### Part N: Preparation of (Z)-Methyl (3-benzylidene-1-carbamoylmethyl-2-ethyl-3H-inden-7-yloxy)-acetate 15

Using the procedure described in the prepartion of compound 13a from 12a. Mp., 129-141 °C. 86% yield.
¹H NMR (300 MHz, CDCl₃) δ 1.24 (3H, t, J=7.5 Hz), 2.72 (2H, q, J=7.5 Hz), 3.81 (2H, s), 3.83 (3H, s), 4.70 (2H, s), 5.24 (1H, br.s), 6.60 (1H, d, J=8.1 Hz), 6.74 (1H, br.s), 6.82 (1H, t J=7.8 Hz), 7.04 (1H, d, J=7.2 Hz), 7.30 (1H, s), 7.31-7.53 (5H, m). IR νₘₐₓ (KBr) 3398, 3174, 2958, 1751, 1684, 1620, 1591, 1573, 1479, 1435, 1390, 1223, 1101, 746, 703 cm⁻¹. Analyses: Calc'd for C₂₃H₂₃NO₄ 0.6H₂O: C, 71.15; H, 6.28; N, 3.61. Found: C, 71.30; H; 6.09; N, 3.69. EIMS m/z=318 (base peak), 377 (M⁺).

### Part O: Preparation of (Z)-Methyl (3-benzyl-1-carbamoylmethyl-2-ethyl-3H-inden-7-yloxy)-acetate 16a and (Z)-methyl (3-benzyl-1-carbamoylmethyl-2-ethyl-1H-inden-7-yloxy)-acetate 16b

A mixture of the acetic acid ester (15, 113 mg, 0.300 m mol) and 5% palladium-coal (11.9 mg) in methanol (5.0 ml) was hydrogenated under atmosphere for 18 min. The catalyst was filtered off and the solvent was evaporated. The residue was purified by preparative TLC on silica gel (200x200x0.25 mm, eluting with AcOEt) to give the crude product (16a, 55.3 mg, 49%) and the isomer (16b, 11.1 mg, 10%). The crude product was recrystallized from CHCl3-hexane to afford the pure product (16a, 48.8 mg, 43%). 16a: Mp., 118-121 °C.

### Part P: Preparation of (Z)-(3-Benzyl-1-carbamoylmethyl-2-ethyl-3H-inden-7-yloxy)-acetic acid 17a

To a solution of the ester (16a, 33.7 mg, 0.0888 m mol) in methanol (1.5 ml) was added 1N sodium hydroxide (0.270 ml, 0.270 m mol) at room temperature. The solution was stirred at this temperature fo 50 min, evaporated, partitioned between 1N HCl and AcOEt. The organic layer was separated, washed with water and brine, dried over MgSO4, filtered and evaporated in vacuo. Recrystallization from CHCl₃-hexane afforded the titled compound (16.7 mg, 50%), mp., 193-197 °C.

¹H NMR (300 MHz, d₆-DMSO) δ 1.05 (3H, t, J=7.5 Hz), 2.16 (1H, dd, J=9.0, 14.7 Hz), 2.23-2.39 (1H, m), 2.53-2.70 (1H, m), 3.06 (1H, dd, J=3.6, 14.7 Hz), 3.82 (2H, s), 3.99 (1H, dd, J=3.2, 8.9 Hz), 4.71 (1H, ABq, Apart J=16.8 Hz), 4.75 (1H, ABq, Bpart J=16.8 Hz), 6.60 (1H, d, J=8.1 Hz), 6.69 (1H, d, J=7.5 Hz), 6.84 (1H, br.s), 7.07 (1H, t, J=7.8 Hz) , 7.10-7.30 (6H, m). IR νₘₐₓ (KBr) 3422, 3225, 2966, 2932, 1718, 1647 1604, 1583, 1475, 1249, 1224, 1097 cm⁻¹. Analyses:
Calc'd for C₂₂H₂₃NO₄ 0.4H₂O: C, 70.91; H, 6.44; N, 3.76. Found: C, 70.79; H; 6.35; N, 3.75. EIMS m/z=231 (base peak), 365 (M⁺).

### Preparation of (Z)-(3-Benzyl-1-carbamoylmethyl-2-ethyl-1H-inden-7-yloxy)-acetic acid 17b

90% Yield. ¹H NMR (300 MHz, d₆-DMSO) δ 1.05 (3H, t, J=7.5 Hz), 2.12-2.37 (2H, m), 2.55-2.70 (1H, m), 3.05 (1H, dd, J=3.3, 14.7 Hz), 3.82 (2H, s), 3.94-4.20 (1H, br m), 4.69 (1H, ABq, Apart J=15.9 Hz), 4.73 (1H, ABq, Bpart J=15.9 Hz), 6.59 (1H, d, J=8.1 Hz), 6.69 (1H, d, J=7.5 Hz), 6.82 (1H, br.s), 7.06 (1H, t, J=7.8 Hz) , 7.10-7.33 (6H, m). EIMS m/z=231 (base peak), 365 (M⁺). HR-EIMS Calc'd for C₂₂H₂₃NO₄: 365.1626, Found:365.1630

### Part O: Preparation of (Z)-2-[3-(Biphenyl-2-ylmethylene)-2-ethyl-7-methoxy-3H-inden-1-yl]-2-hydroxy-acetic acid 18

To a solution of diisopropylamine (0.860 ml, 6.14 m mol) in THF (25.0 ml) was added n-butyllithium (1.6 M in hexane solution, 4.40 ml) at -20°C. After the reaction mixture was stirred for 20 min at -20°C and for 20 min at 0°C, a solution of acetic acid derivative (10d, 0.501 g, 1.26 m mol) in THF (35 ml) was slowly added at -78°C. The mixture was gradually warmed to 0°C, was bubbled with air for 10 min at this temperature and then was poured into 1N HCl and extracted with AcOEt. The extracts was washed with saturated Na₂S₂O₃ solution, water and brine, dried over MgSO₄, filtered and concentrated in vacuo. The residue was roughly purified by preparative TLC on silica gel (200x200x2 mm, elution with CHCl₃:MeOH=95:5) to give the crude product (0.333 g). The crude product was used the next preparation without furter purification.

### Part R: Preparation of (Z)-2-[3-(Biphenyl-2-ylmethylene)-2-ethyl-7-methoxy-3H-inden-1-yl]-2-hydroxy-acetamide 19

Using the procedure described in the prepartion of compound 11a from 10, the hydroxy acetic acid (18, 0.286 g, 0.694 m mol) in acetonitrile (10 ml) was reacted with benzotriazol-1-yloxytris(dimethylamino) phosphonium hexafuluorophoshate (0.371 g, 0.839 m mol), triethylamine (0.120 ml, 0.861 m mol) and 28% aqueous ammonia (0.470 ml, 6.95 m mol) to give 100 mg (2 steps yield=22%) of the titled compound, mp., 204-209 °C.

### Part S: Preparation of (Z)-2-[3-(Biphenyl-2-ylmethylene)-2-ethyl-7-methoxy-3H-inden-1-yl]-2-oxo-acetamide 20

To a solution of the hydroxy acetamide (19, 49.3 mg, 0.120 m mol) in dichloromethane (1.5 ml) were added molecular sieves (4A powder, 61.3 mg), N-methylmorpholine N-oxide (57.3 mg, 0.489 m mol) and tetrapropylammonium perruthenate (4.3 mg, 0.0112 m mol) at room temperature and then the reaction mixture was subjected successively to chromatography on silica gel eluting with CHCl₃ and then CHCl₃:MeOH=9:1, to prepreparative TLC on silica gel (200x200x2 mm), eluting with CHCl₃:MeOH=97.5:2.5) and recrystallization from hexane-CHCl₃ to afford 27.2 mg (55%) of the desired product, mp., 172-179 °C.

¹H NMR (200 MHz, CDCl₃) δ 1.02 (3H, t, J=7.6 Hz), 2.47 (2H, q, J=7.6 Hz), 3.80 (3H, s), 5.60 (1H, br.s), 6.73 (1H, br.s), 6.76 (1H, d, J=8.2 Hz), 6.96 (1H, t, J=7.8 Hz), 7.18 (1H, s), 7.24 (1H, d, J=7.6 Hz), 7.30-7.66 (8H, m), 7.91 (1H, d, J=7.4 Hz). IR νₘₐₓ (KBr) 3437, 3211, 2968, 2934, 1665, 1624, 1600, 1568, 1482, 1341, 1274, 1267, 1152, 1066, 748, 701 cm⁻¹. Analyses: Calc'd for C₂₇H₂₃NO₃ 0.5H₂O: C, 77.49 H, 5.78; N, 3.35. Found: C, 77.35; H; 5.86; N, 3.41. EIMS m/z=165 (base peak), 409 (M+).

### Therapeutic Use of indene-1-functional compounds

The indene-1-functional compounds of the invention are believed to achieve their beneficial therapeutic action principally by direct inhibition of human sPLA₂, and not by acting as antagonists for arachidonic acid, nor other active agents below arachidonic acid in the arachidonic acid cascade, such as 5-lipoxygenases, cyclooxygenases, and etc.

The invention also comprises the use of a therapeutically effective amount of a compound as defined in the claims or a salt or a prodrug derivative thereof for the manufacture of a medicament for inhibiting sPLA₂ mediated release of fatty acids in a mammal, including a human, suffering from or susceptible to a disease in which sPLA₂ mediated release of fatty acids is a cause.

The compounds of the invention may be used for the manufacture of a medicament for treating a mammal (e.g., a human) to alleviate the pathological effects of septic shock, adult respiratory distress syndrome, pancreatitis, trauma, bronchial asthma, allergic rhinitis, and rheumatoid arthritis; wherein the medicament comprises at least one compound as defined in the claims or any combination thereof in a therapeutically effective amount. A therapeutically effective amount is an amount sufficient to inhibit sPLA₂ mediated release of fatty acid and to thereby inhibit or prevent the arachidonic acid cascade and its deleterious products. The therapeutic amount of compound of the invention needed to inhibit sPLA₂ may be readily determined by taking a sample of body fluid and assaying it for sPLA₂ content by conventional methods.

### Pharmaceutical Formulations of the Invention

As previously noted the compounds of this invention are useful for inhibiting sPLA₂ mediated release of fatty acids such as arachidonic acid. By the term, "inhibiting" is meant the prevention or therapeutically significant reduction in release of sPLA₂ initiated fatty acids by the compounds of the invention. By "pharmaceutically acceptable" it is meant the carrier, diluent or excipient must be compatible with the other ingredients of the formulation and not deleterious to the recipient thereof.

The specific dose of a compound administered according to this invention to obtain therapeutic or prophylactic effects will, of course, be determined by the particular circumstances surrounding the case, including, for example, the compound administered, the route of administration and the condition being treated. Typical daily doses will contain a non-toxic dosage level of from 0.01 mg/kg to 50 mg/kg of body weight of an active compound of this invention.

Preferably the pharmaceutical formulation is in unit dosage form. The unit dosage form can be a capsule or tablet itself, or the appropriate number of any of these. The quantity of active ingredient in a unit dose of composition may be varied or adjusted from 0.1 to 1000 milligrams or more according to the particular treatment involved. It may be appreciated that it may be necessary to make routine variations to the dosage depending on the age and condition of the patient. The dosage will also depend on the route of administration.

The compound can be administered by a variety of routes including oral, aerosol, rectal, transdermal, subcutaneous, intravenous, intramuscular, and intranasal.

Pharmaceutical formulations of the invention are prepared by combining (e.g., mixing) a therapeutically effective amount of the indene-1-functional compounds of the invention together with a pharmaceutically acceptable carrier or diluent therefor. The present pharmaceutical formulations are prepared by known procedures using well known and readily available ingredients.

In making the compositions of the present invention, the active ingredient will usually be admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, or can be in the form of tablets, pills, powders, lozenges, elixirs, suspensions, emulsions, solutions, syrups, aerosols (as a solid or in a liquid medium), or ointment, containing, for example, up to 10% by weight of the active compound.

The compounds of the present invention are preferably formulated prior to administration.

For the pharmaceutical formulations any suitable carrier known in the art can be used. In such a formulation, the carrier may be a solid, liquid, or mixture of a solid and a liquid. Solid form formulations include powders, tablets and capsules. A solid carrier can be one or more substances which may also act as flavoring agents, lubricants, solubilisers, suspending agents, binders, tablet disintegrating agents and encapsulating material.

Tablets for oral administration may contain suitable excipients such as calcium carbonate, sodium carbonate, lactose, calcium phosphate, together with disintegrating agents, such as maize, starch, or alginic acid, and/or binding agents, for example, gelatin or acacia, and lubricating agents such as magnesium stearate, stearic acid, or talc.

In powders the carrier is a finely divided solid which is in admixture with the finely divided active ingredient. In tablets the active ingredient is mixed with a carrier having the necessary binding properties in suitable proportions and compacted in the shape and size desired. The powders and tablets preferably contain from 1 to 99 weight percent of the active ingredient which is the novel compound of this invention. Suitable solid carriers are magnesium carbonate, magnesium stearate, talc, sugar lactose, pectin, dextrin, starch, gelatin, tragacanth, methyl cellulose, sodium carboxymethyl cellulose, low melting waxes, and cocoa butter.

Sterile liquid form formulations include suspensions, emulsions, syrups and elixirs.

The active ingredient can be dissolved or suspended in a pharmaceutically acceptable carrier, such as sterile water, sterile organic solvent or a mixture of both. The active ingredient can often be dissolved in a suitable organic solvent, for instance aqueous propylene glycol. Other compositions can be made by dispersing the finely divided active ingredient in aqueous starch or sodium carboxymethyl cellulose solution or in a suitable oil.

"Active ingredient", refers to a compound as defined in the claims or a pharmaceutically acceptable salt, solvate, or prodrug derivative thereof.

### Formulation 1

Hard gelatin capsules are prepared using the following ingredients:

| | Quantity (mg/capsule) |
|---|---|
| Active ingredient | 250 |
| Starch, dried | 200 |
| Magnesium stearate | 10 |
| Total | $\overline{\text{460 mg}}$ |

### Formulation 2

A tablet is prepared using the ingredients below:

| | Quantity (mg/tablet) |
|---|---|
| Active ingredient | 250 |
| Cellulose, microcrystalline | 400 |
| Silicon dioxide, fumed | 10 |
| Stearic acid | 5 |
| Total | $\overline{\text{665 mg}}$ |

The components are blended and compressed to form tablets each weighing 665 mg

### Formulation 3

An aerosol solution is prepared containing the following components:

| | Weight |
|---|---|
| Active ingredient | 0.25 |
| Ethanol | 25.75 |
| Propellant 22 (Chlorodifluoromethane) | 74.00 |
| Total | $\overline{\text{100.00}}$ |

The active compound is mixed with ethanol and the mixture added to a portion of the propellant 22, cooled to -30°C and transferred to a filling device. The required amount is then fed to a stainless steel container and diluted with the remainder of the propellant. The valve units are then fitted to the container.

### Formulation 4

Tablets, each containing 60 mg of active ingredient, are made as follows:

| | |
|---|---|
| Active ingredient | 60 mg |
| Starch | 45 mg |
| Microcrystalline cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | $\overline{\text{150 mg}}$ |

The active ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

### Formulation 5

Capsules, each containing 80 mg of active ingredient, are made as follows:

| | |
|---|---|
| Active ingredient | 80 mg |
| Starch | 59 mg |
| Microcrystalline cellulose | 59 mg |
| Magnesium stearate | 2 mg |
| Total | $\overline{\text{200 mg}}$ |

The active ingredient, cellulose, starch, and magnesium stearate are blended, passed through a No. 45 mesh U.S. sieve, and filled into hard gelatin capsules in 200 mg quantities.

### Formulation 6

Suppositories, each containing 225 mg of active ingredient, are made as follows:

| | |
|---|---|
| Active ingredient | 225 mg |
| Saturated fatty acid glycerides | 2,000 mg |
| Total | $\overline{\text{2,225 mg}}$ |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

### Formulation 7

Suspensions, each containing 50 mg of active ingredient per 5 ml dose, are made as follows:

| | |
|---|---|
| Active ingredient | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water to total | 5 ml |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

### Formulation 8

An intravenous formulation may be prepared as follows:

| | |
|---|---|
| Active ingredient | 100 mg |
| Isotonic saline | 1,000 ml |

The solution of the above ingredients generally is administered intravenously to a subject at a rate of 1 ml per minute.

### Assay Experiments

The following chromogenic assay procedure was used to identify and evaluate inhibitors of recombinant human secreted phospholipase A₂. The assay described herein has been adapted for high volume screening using 96 well microtiter plates. A general description of this assay method is found in the article, "Analysis of Human Synovial Fluid Phospholipase A₂ on Short Chain Phosphatidylcholine-Mixed Micelles: Development of a Spectrophotometric Assay Suitable for a Microtiterplate Reader", by Laure J. Reynolds, Lori L. Hughes, and Edward A Dennis, Analytical Biochemistry, 204, pp. 190-197, 1992. Reagents:

| REACTION BUFFER - | |
|---|---|
| CaCl₂.2H₂O | (1.47 g/L) |
| KCl | (7.455 g/L) |
| Bovine Serum Albumin (fatty acid free) (1 g/L) (Sigma A-7030, product of Sigma Chemical Co. St. Louis MO, USA) | |
| TRIS HCl | (3.94 g/L) |
| pH 7.5 (adjust with NaOH) | |

| ENZYME BUFFER - | |
|---|---|
| 0.05 NaOAc.3H₂O, pH 4.5 | |
| 0.2 NaCl | |
| Adjust pH to 4.5 with acetic acid | |
| DTNB - 5,5'-dithiobis-2-nitrobenzoic acid | |

| RACEMIC DIHEPTANOYL THIO - PC | |
|---|---|
| racemic 1,2-bis(heptanoylthio)-1,2-dideoxy-*sn*-glycero-3-phosphorylcholine | |
| TRITON X-100™ prepare at 6.249 mg/ml in reaction buffer to equal 10µM. | |

| REACTION MIXTURE - | |
|---|---|
| | |

A measured volume of racemic dipheptanoyl thio PC supplied in chloroform at a concentration of 100 mg/ml is taken to dryness and redissolved in 10 millimolar TRITON X-100™ nonionic detergent aqueous solution. Reaction Buffer is added to the solution, then DTNB to give the Reaction Mixture.

The reaction mixture thus obtained contains 1mM diheptanoly thio-PC substrate, 0.29 mm Triton X-100™ detergent, and 0.12 mm DTMB in a buffered aqueous solution at pH 7.5.

### Assay Procedure:

1. Add 0.2 ml reaction mixture to all wells;
2. Add 10 µl test compound (or solvent blank) to appropriate wells, mix 20 seconds;
3. Add 50 nanograms of sPLA₂ (10 microliters) to appropriate wells;
4. Incubate plate at 40°C for 30 minutes;
5. Read absorbance of wells at 405 nanometers with an automatic plate reader.

All compounds were tested in triplicate. Typically, compounds were tested at a final concentration of 5 µg/ml. Compounds were considered active when they exhibited 40% inhibition or greater compared to uninhibited control reactions when measured at 405 nanometers. Lack of color development at 405 nanometers evidenced inhibition. Compounds initially found to be active were reassayed to confirm their activity and, if sufficiently active, IC₅₀ values were determined. Typically, the IC₅₀ values (see, Table I, below) were determined by diluting test compound serially two-fold such that the final concentration in the reaction ranged from 45 µg/mL to 0.35 µg/ml. More potent inhibitors required significantly greater dilution. In all cases, % inhibition measured at 405 nanometers generated by enzyme reactions containing inhibitors relative to the uninhibited control reactions was determined. Each sample was titrated in triplicate and result values were averaged for plotting and calculation of IC₅₀ values. IC₅₀ were determined by plotting log concentration versus inhibition values in the range from 10-90% inhibition.

The results of Human Secreted Phospholipase A₂ Inhibition tests are displayed in the Table below:

**TABLE**

| sPLA₂ Chromogenic Assay Data | |
|---|---|
| 14a | 0.91 |
| 14b | 0.42 |
| 14c | 0.16 |
| 14d | 0.10 |
| 14e | 0.40 |
| 14f | 0.20 |
| 14g | 0.067 |
| 14h | 0.24 |
| 14i | 0.025 |
| 14j | 0.17 |
| 17a | 15 |
| 17b | 12 |

## Claims

1. An indene-1-acetamide compound or a pharmaceutically acceptable salt, solvate or prodrug derivative thereof; wherein said compound is selected from the group represented by formulae 14a, 14b, 14c, 14d, 14e, 14f, 14g, 14h, 14i, 14j, 17a, and 17b as follows: 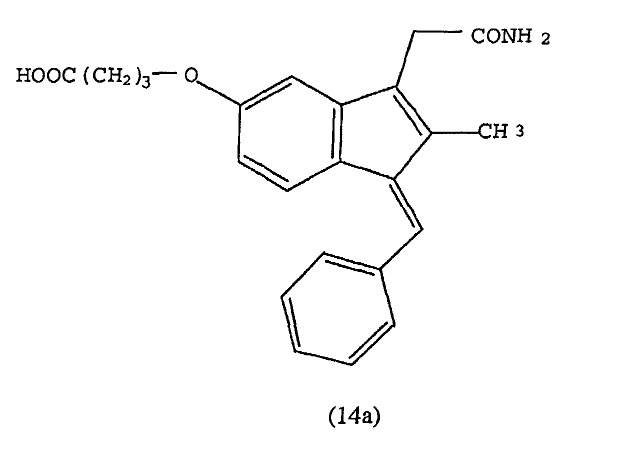 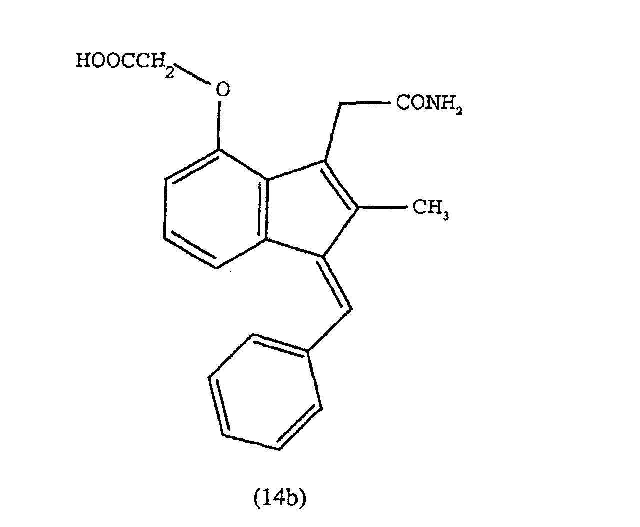 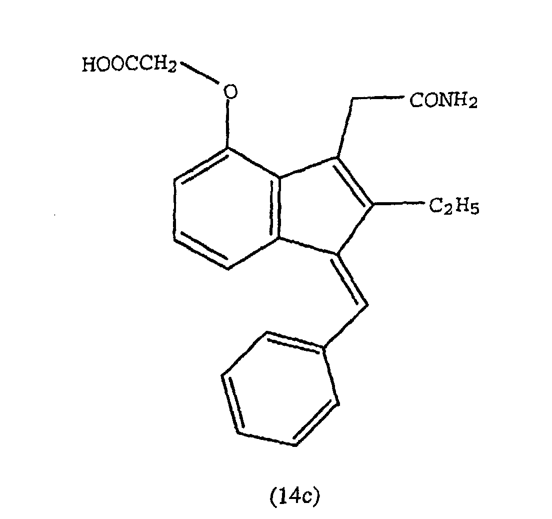 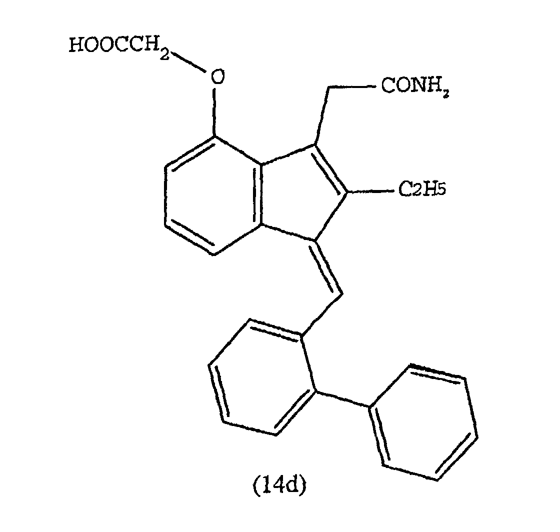 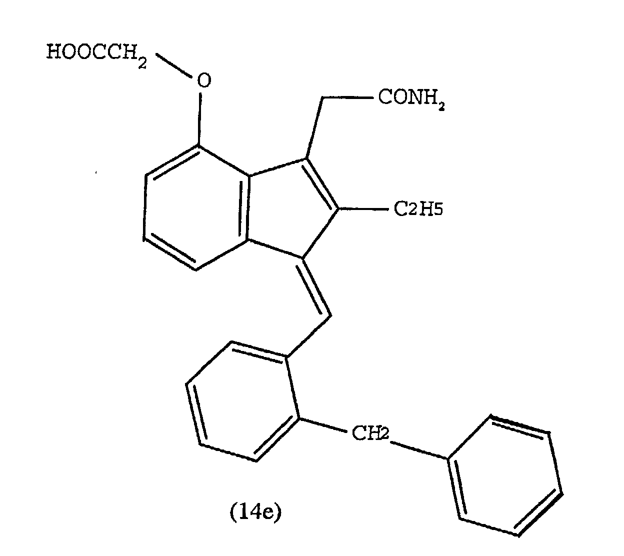 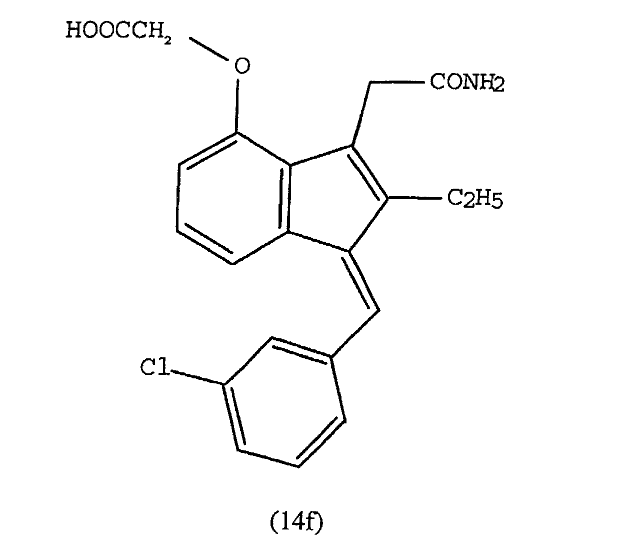 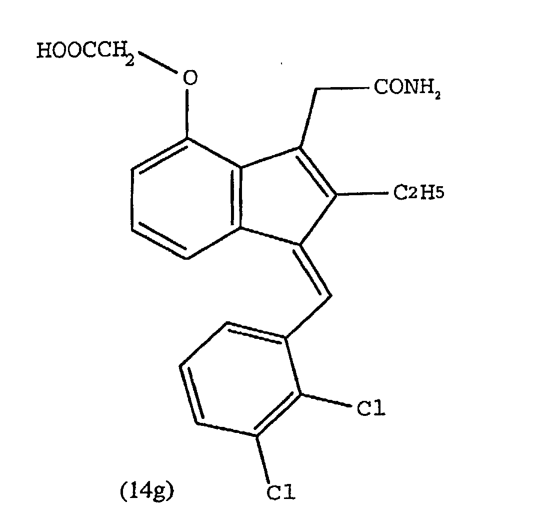 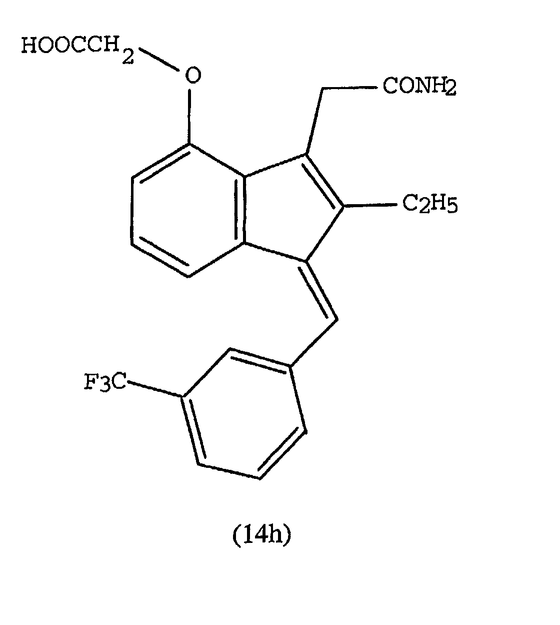 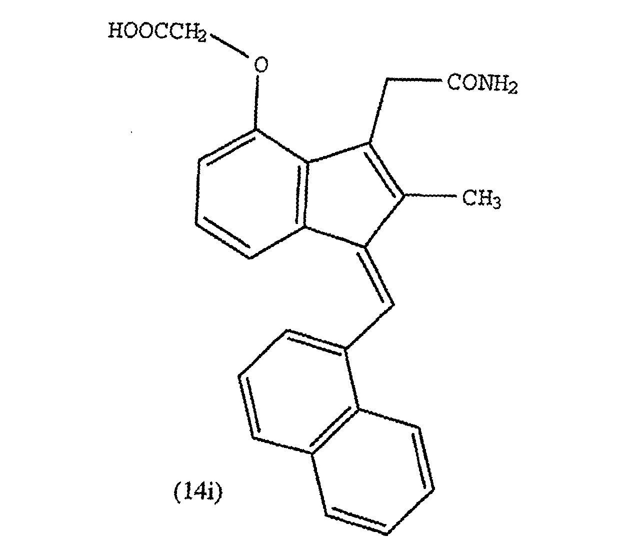 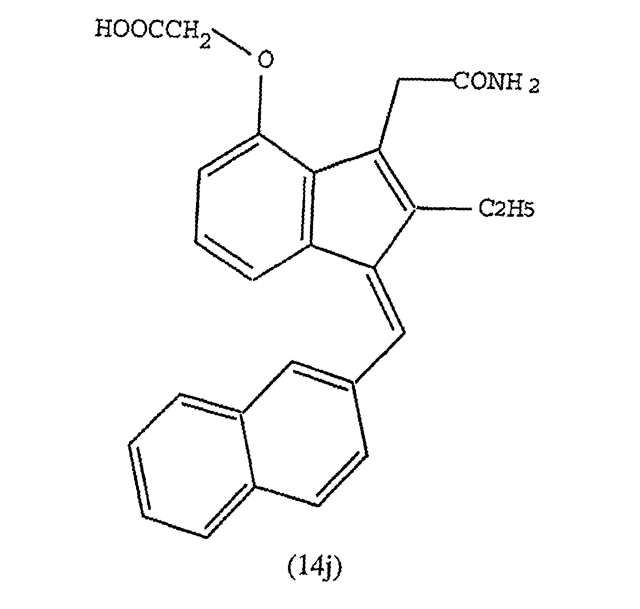 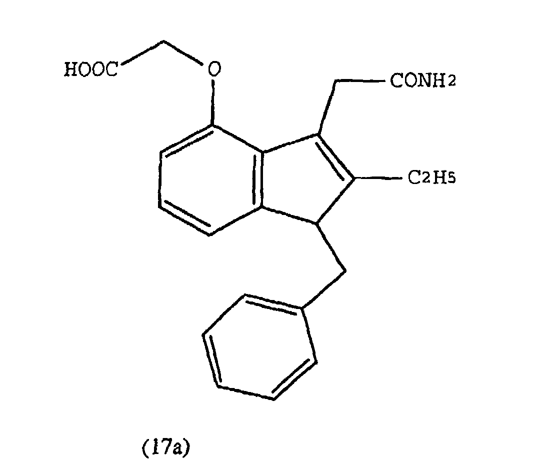 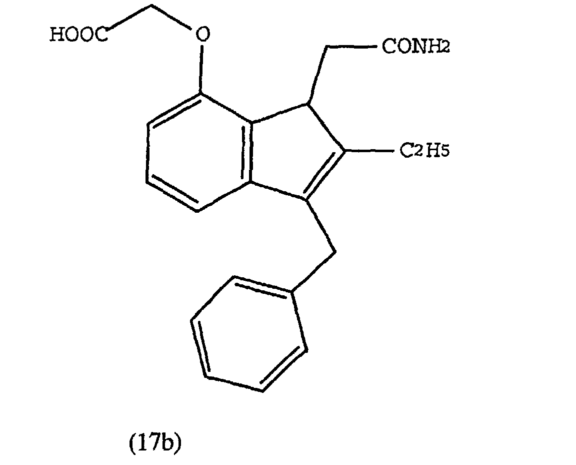 and mixtures thereof.

2. A pharmaceutical formulation comprising the indene-1-acetamide as claimed in Claim 1 together with a pharmaceutically acceptable carrier or diluent therefor.

3. Use of a compound as claimed in Claim 1 for the manufacture of a medicament for the treatment of septic shock, adult respiratory distress syndrome, pancreatitis, trauma, bronchial asthma, allergic rhinitis, and rheumatoid arthritis.

## Patentansprüche

1. Indol-1-acetamidverbindung oder ein pharmazeutisch annehmbares Salz, Solvat oder Prodrugderivat hiervon, worin die Verbindung ausgewählt ist aus der Gruppe, die folgendennaßen durch die Formeln 14a, 14b, 14c, 14d, 14e, 14f, 14g, 14h, 14i, 14j, 17a und 17b dargestellt wird: 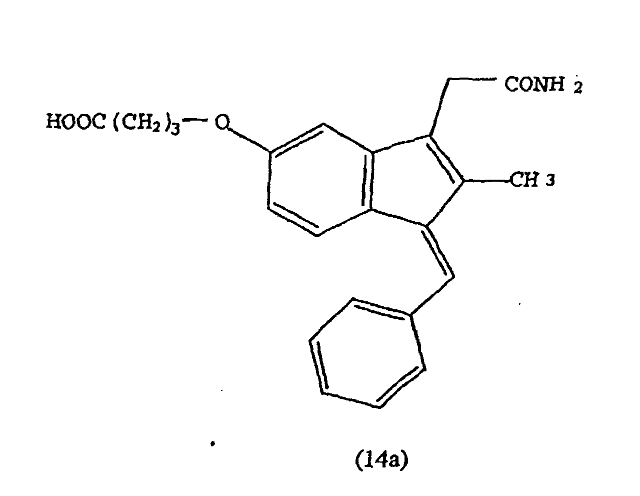 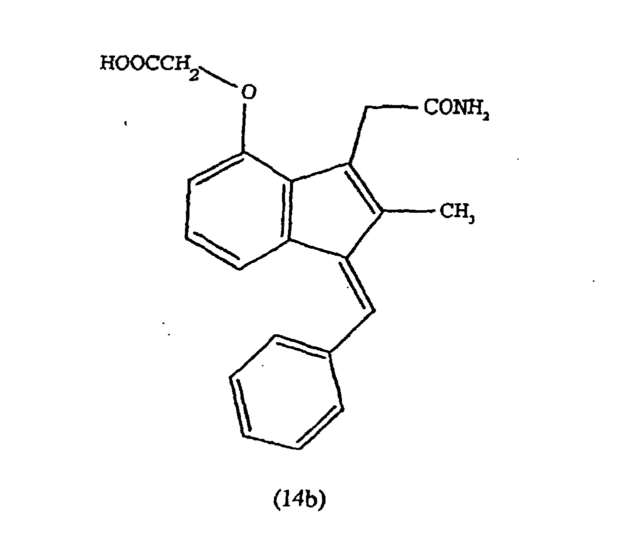 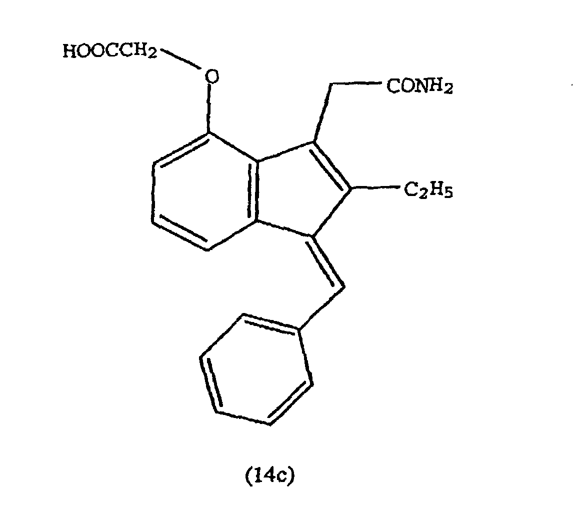 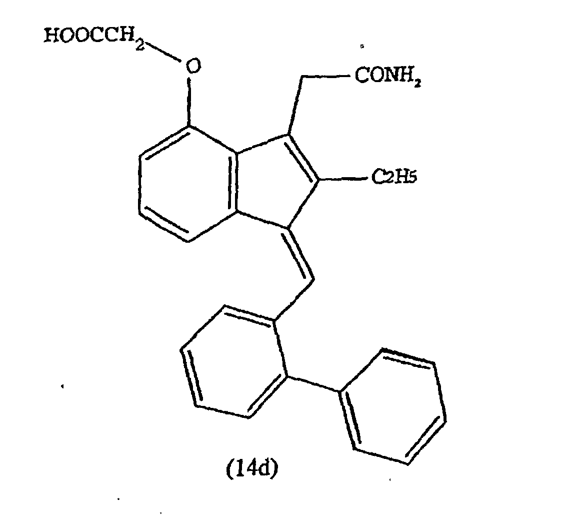 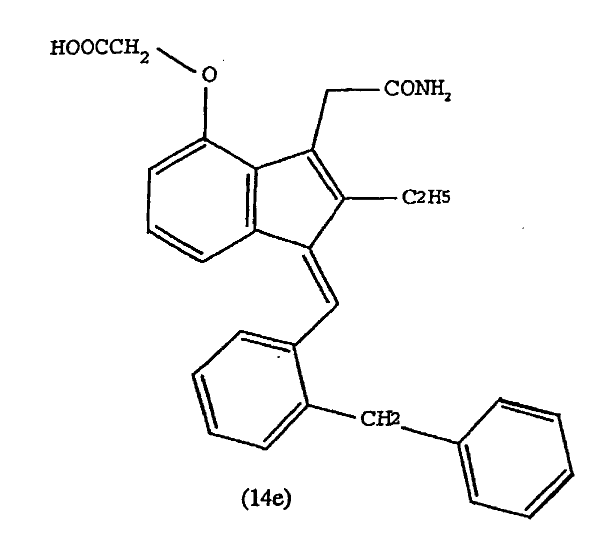 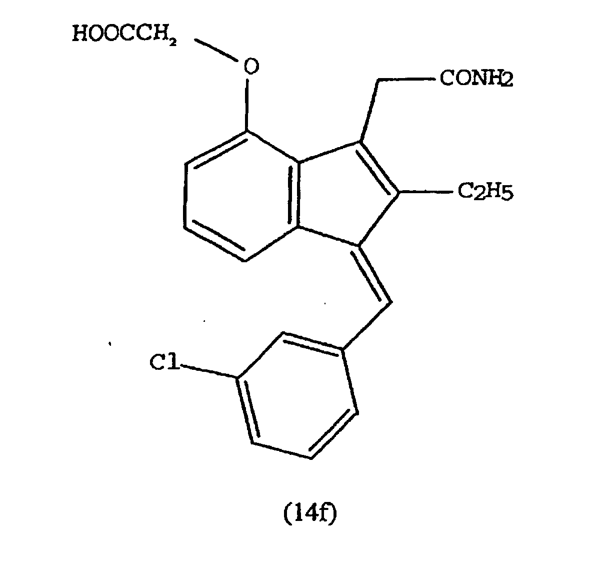 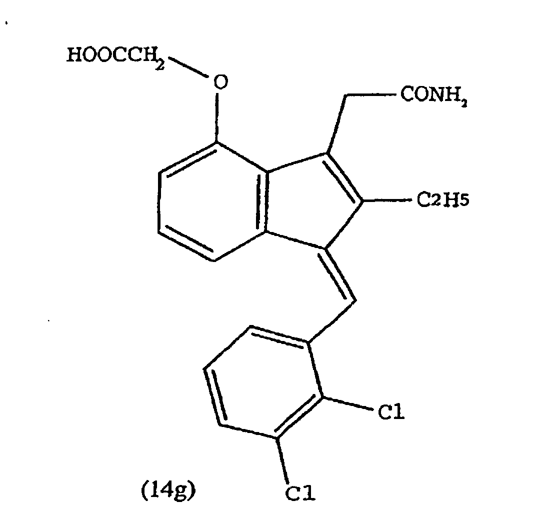 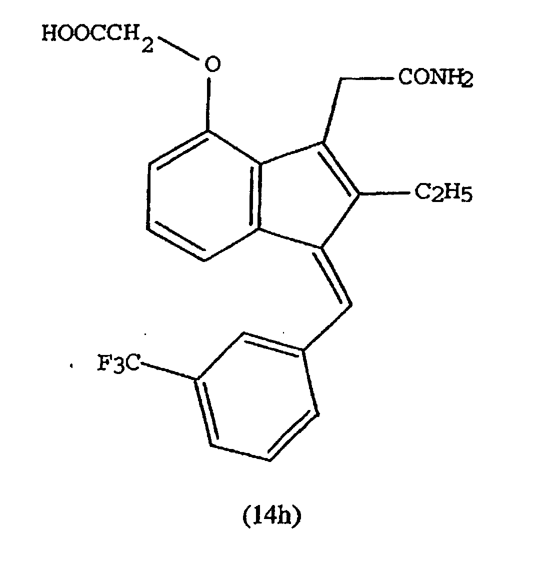 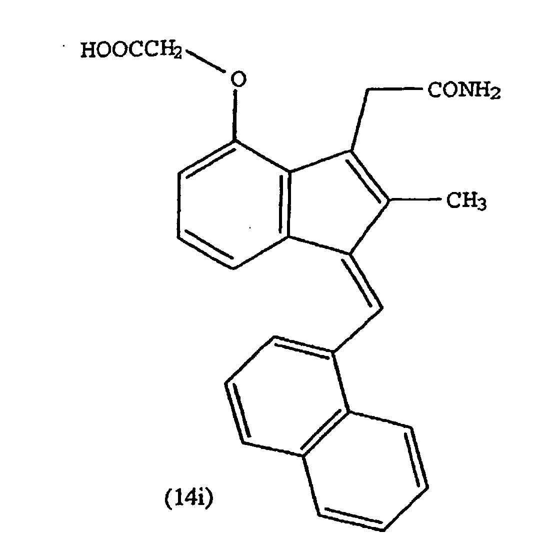 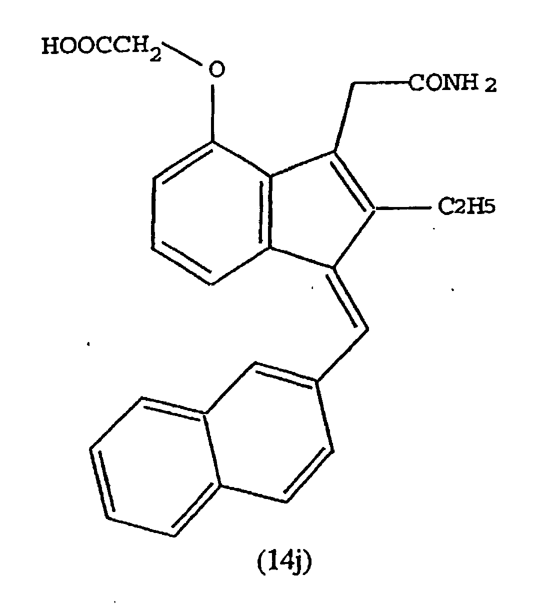 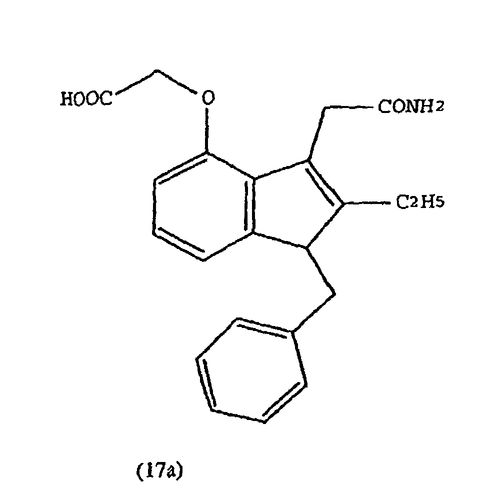 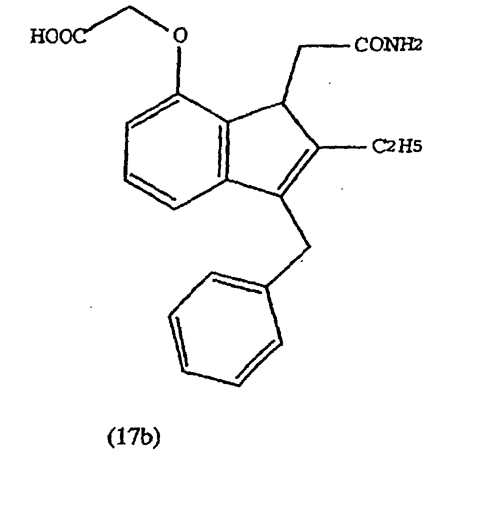 und Gemische hiervon.

2. Pharmazeutische Formulierung, die das Indol-1-acetamid nach Anspruch 1 zusammen mit einem pharmazeutischen Träger oder Verdünnungsmittel hierfür enthält.

3. Verwendung einer Verbindung nach Anspruch 1 zur Herstellung eines Arzneimittels zur Behandlung von septischem Schock, Atemstresssyndrom beim Erwachsenen, Pankreatitis, Trauma, Bronchialasthma, allergischer Rhinitis und rheumatoider Arthritis.

## Revendications

1. Composé d'indène-1-acétamide ou un de ses sels, solvates, ou dérivés de promédicament, pharmaceutiquement acceptables ; dans lequel ledit composé est choisi dans le groupe représenté par les formules 14a, 14b, 14c, 14d, 14e, 14f, 14g, 14h, 14i, 14j, 17a, et 17b comme suit : 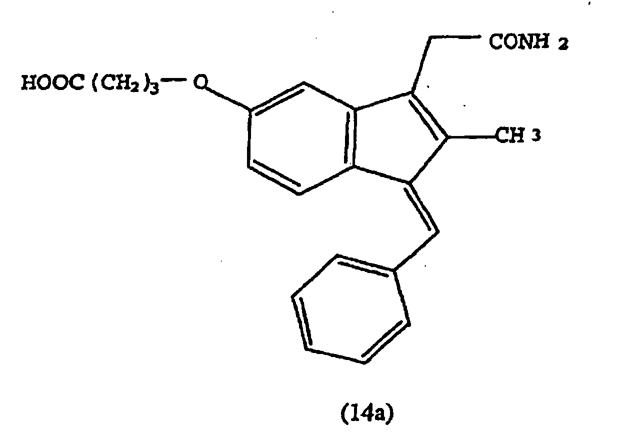 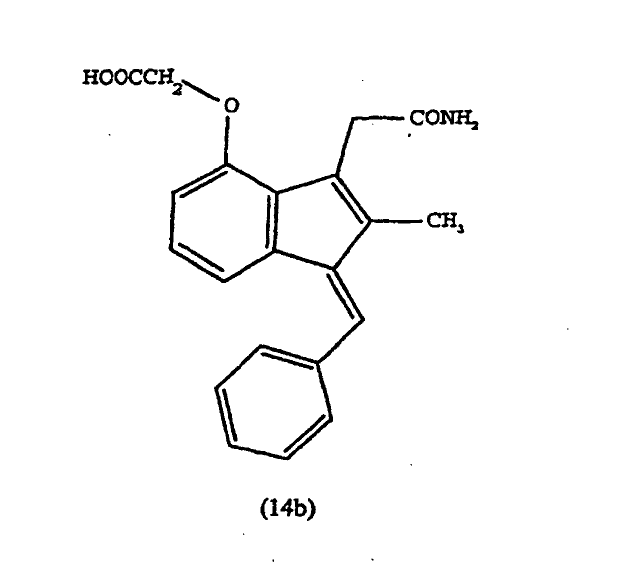 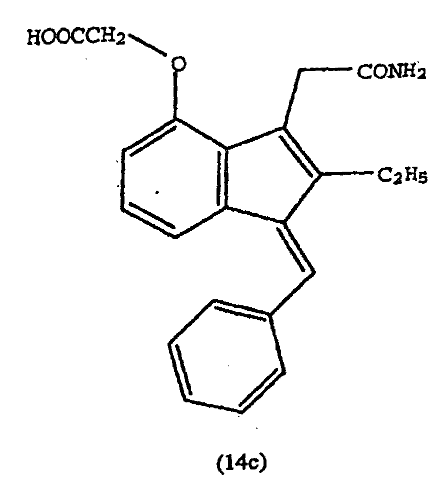 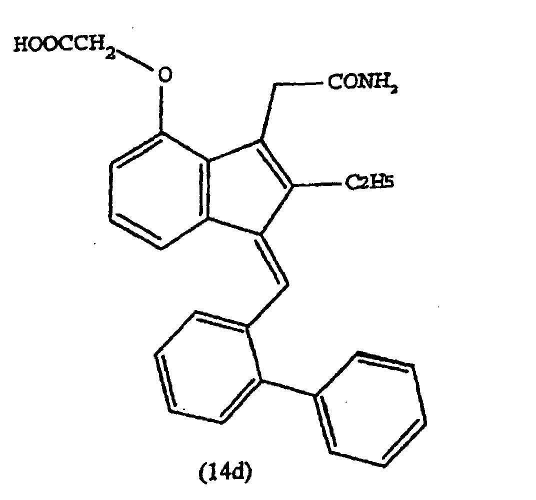 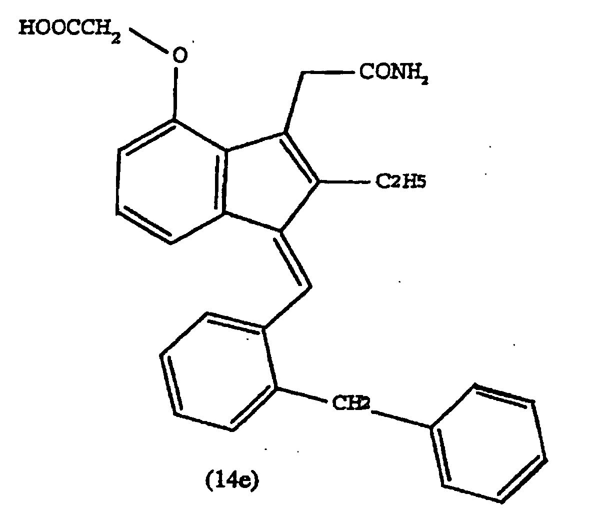 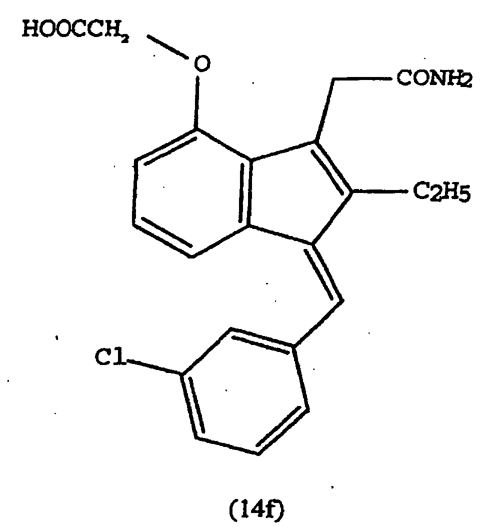 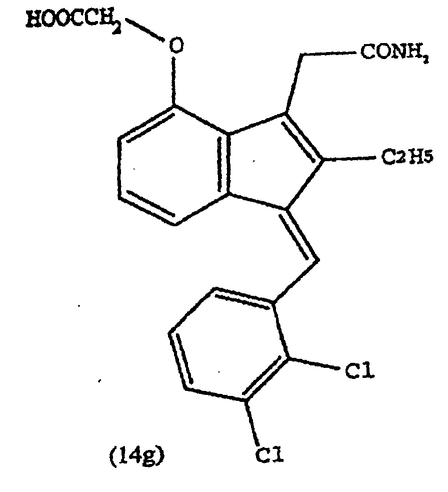 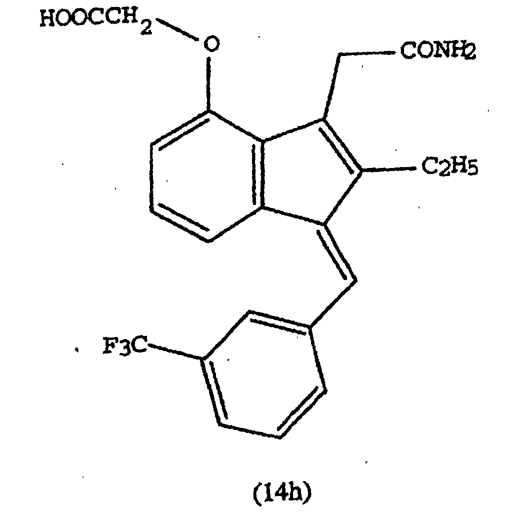 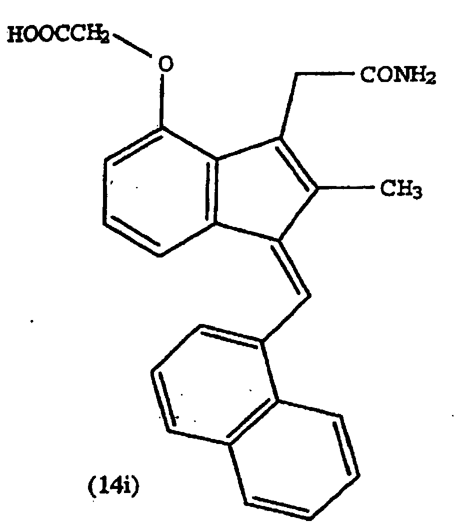 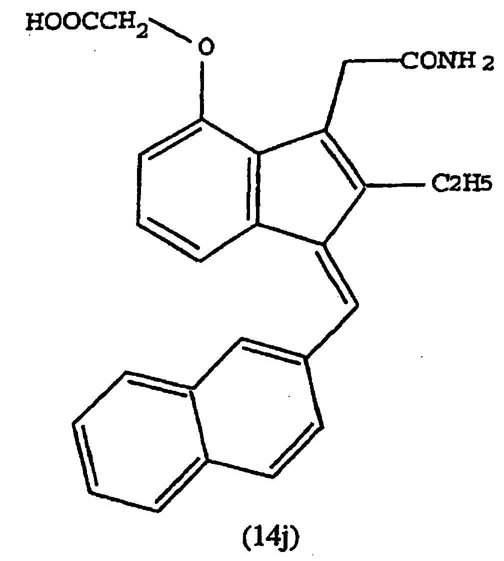 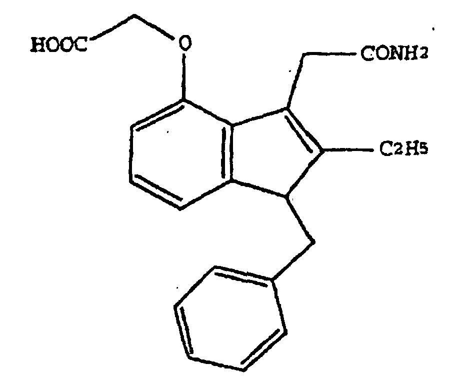 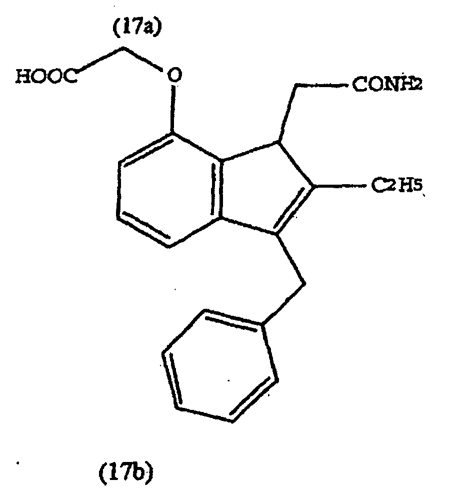 et les mélanges de ceux-ci.

2. Formulation pharmaceutique comprenant le composé d'indène-1-acétamide selon la revendication 1 conjointement avec un support ou diluant pour celui-ci pharmaceutiquement acceptable.

3. Utilisation d'un composé selon la revendication 1 pour la fabrication d'un médicament destiné au traitement du choc septique, du syndrome de détresse respiratoire chez l'adulte, de la pancréatite, du traumatisme, de l'asthme bronchique, de la rhinite allergique, et de la polyarthrite rhumatoïde.
